# EUROPÄISCHE PATENTANMELDUNG

(11) **EP 1 728 519 A2**
(43) Veröffentlichungstag der Anmeldung: **06.12.2006**
(21) Anmeldenummer: 06114870.6
(22) Anmeldetag: 01.06.2006
(51) Int. Cl.: A61K 41/00, A61H 39/00

(54) **Polares Unterstützungs- und Breitbandresonatorpräparat**

(30) Priorität: 01.06.2005 DE 102005025156
(71) Anmelder: Natterer, Siegfried, 92637 Weiden (DE)
(72) Erfinder: Natterer, Siegfried, 92637 Weiden (DE)
(74) Vertreter: Lang, Christian

(57) **Zusammenfassung**

Die vorliegende Erfindung betrifft ein Präparat zur Unterstützung der Aufnahme von arzneilichen Wirkstoffen oder Nahrungsergänzungsmitteln in einem menschlichen oder tierischen Körper, wobei mindestens eine polare Trägersubstanz und mindestens ein Breitbandresonator für den menschlichen oder tierischen Körper enthalten sind, sowie ein Präparat zur Vermeidung toxischer Wirkungen von Substanzen, die auf den menschlichen oder tierischen Körper einwirken, und zwar durch Beimischung mindestens eines Breitbandresonators. Ferner werden ein Verfahren zur Herstellung der Präparate und ein Mehrkomponentenpräparat vorgestellt.

## Beschreibung

Die vorliegende Erfindung betrifft ein Präparat zur Aktivierung der Enzymsysteme biologischer Systeme und zur Unterstützung der Aufnahme und/oder Steigerung der Wirkung von insbesondere arzneilichen Wirkstoffen oder Nahrungsergänzungsmitteln und/oder zur Vermeidung oder Verringerung toxischer Wirkungen von Substanzen, ein Mehrstufenpräparat sowie ein Präparate-Set und ein Verfahren zu deren Herstellung sowie die Anwendung derartiger Präparate.

### STAND DER TECHNIK

Die moderne Medizin stellt eine Vielzahl von Arzneimitteln und Pharmazeutika bereit, die sowohl auf synthetisch hergestellten Substanzen beruhen als auch auf natürliche Wirkstoffe, wie pflanzliche und tierische Wirkstoffe zurückgreifen. Hierbei ist festzustellen, dass oftmals die Wirkstoffe bei unterschiedlichen Patienten sehr unterschiedliche Wirkungen zeigen. Während sie bei bestimmten Patienten positive Wirkungen mit einer Verbesserung des Krankheitsbildes bewirken, sind bei anderen Patienten mit einem vergleichbaren Krankheitsbild keine Wirkungen oder sogar überwiegend negative Wirkungen, so genannte Nebenwirkungen festzustellen.

Dies führt dazu, dass in Einzelfällen oftmals Medikamente, die bei einem überwiegenden Teil von Patienten zu einer Heilung führen, bei anderen Anwendern zu schwerwiegenden Nebenwirkungen und/oder zu mangelnder Wirksamkeit des Präparats führen. Außer bei Medikamenten können derartige Probleme hinsichtlich der Wirkung oder Verträglichkeit auch bei Nahrungsergänzungsmitteln oder anderen Stoffen, die auf den Körper einwirken, wie Kosmetika und dgl., auftreten. So können beispielsweise Kosmetika bei einzelnen Anwendern zu schweren Unverträglichkeiten führen, die bei anderen Anwendern nicht zu beobachten sind. Dabei gilt das Prinzip der Dosis- Wirkung- Beziehung. So geht man davon aus, dass eine Erhöhung der Dosis nicht nur die Wirkung verstärkt, sondern gleichzeitig auch Nebenwirkungen stärker hervortreten.

### AUFGABE DER ERFINDUNG

Es ist deshalb Aufgabe der vorliegenden Erfindung für die oben beschriebenen Probleme eine Lösung bereit zu stellen und für Abhilfe zu sorgen. Insbesondere soll vorzugsweise mittels eines oder mehrerer Hilfsstoffe die Resorption, Verträglichkeit und Wirksamkeit von Lebensmitteln/Nahrungsergänzungmitteln, pharmazeutischen Präparaten, Arzneimitteln aller Art und sonstigen von Menschen oder Tieren aufgenommenen oder mit ihnen in Kontakt kommenden Stoffen insbesondere im Sinne einer Verringerung des toxischen und allergenen Potentials verbessert werden.

### WESEN DER ERFINDUNG

Diese Aufgabe wird gelöst mit einem Präparat mit den Merkmalen des Ansprüche 1, 2 und 4, einem Mehrstufenpräparat mit den Merkmalen des Anspruchs 42, einem Präparate-Set mit den Merkmalen des Anspruchs 44 und einem Verfahren zur Herstellung der Präparate mit den Merkmalen des Anspruchs 47 sowie der Verwendung der Präparate nach den Ansprüchen 60 und 61. Vorteilhafte Ausgestaltungen sind Gegenstand der abhängigen Ansprüche.

Die Erfindung geht aus von der Erkenntnis, dass Wirkstoffe oder Substanzen, die auf den menschlichen oder tierischen Körper einwirken oder von diesem aufgenommen werden, in Resonanz mit dem biologischen System des Körpers gehen müssen, um ihre positive Wirkung zu entfalten. Ist die Resonanz gestört, so kann der Stoff keine Wirkung entfalten. Bei Medikamenten oder Nahrungsergänzungsmitteln bedeutet dies, dass sie nicht entsprechend wirken, während bei schädlichen, wie toxischen oder allergenen Stoffen die nachteiligen Wirkungen vermindert werden oder nicht auftreten. Somit muss für positiv wirkende Stoffe, wie Arzneimittel, die Resonanz hergestellt, für negativ wirkende Stoffe, wie Allergene, die Resonanz unterdrückt werden. Allerdings besitzen Medikamente oder arzneiliche Wirkstoffe oftmals nicht nur positive Wirkungen, sondern auch negative Nebenwirkungen. In diesem Fall kommt es darauf an Resonanz für die positive Wirkung bereit zu stellen, aber die negative Wirkung gleichzeitig zu dämpfen.

Ferner besteht bei Kranken oftmals die Problematik, dass das biologische System derart gestört ist, dass für die Aufnahme des positiven Wirkstoffs die erforderliche Resonanz nicht hergestellt werden kann.

Die Erfindung geht somit vom Wellenmodell geht aus, nach welchen Wirkstoffe oder Substanzen, die auf den menschlichen oder tierischen Körper einwirken oder von diesem aufgenommen werden, in Resonanz mit dem bioenergetischen Feld und den Strukturen des Körpers gehen müssen, um ihre positive Wirkung zu entfalten. Ist die Resonanz gestört, so kann der Stoff keine Wirkung entfalten.

Somit beruhen sämtliche biologischen, chemischen und pharmakologischen Wirkungen letztlich auf Schwingungen/Wellen und deren Wechselwirkungen und deshalb besitzen zur Optimierung von Präparaten oder Formulierungen die physikalischen und chemischen Eigenschaften die entscheidende Bedeutung. Diese sind nicht als Gegensatz zum biochemischen Modell zu betracheten, sondern schließen quasi mechanische Vorstellungen wie beispielsweise die der Enzymtheorie (Schlüssel-Schloss-Prinzip) oder der Pharmakologie (Rezeptormodell) mit ein, die als Grenzfall des Wellenmodells aufgefasst werden können. Da es sich hier um ein grundlegendes und universelles Prinzip handelt besitzt dieses Gültigkeit für alle Formen der Wirksamkeit von Stoffen auf den Organismus. Entsprechend sind die Übergänge von der Physik zur Chemie naturgemäß fließend, so dass die die Erfindung kennzeichnenden Parameter sowohl mit physikalischen wie chemischen Begriffen beschrieben werden können. Mittels Resonanzwirkungen ist eine selektive Übertragung von Wirkungen über Entfernungen möglich und so werden sowohl lokale Effekte wie auch nicht nicht-lokale Wirkungen erreicht.

Aus Sicht des Schwingungsmodells lässt sich der biologische Organismus als ein Detektorsystem auffassen, das bei der Verarbeitung von Reizen vor die Aufgabe gestellt ist, informative Schwingungen (Signale) von Schwingungen ohne Informationen (Thermisches Rauschen) zu trennen. Dabei gelingt es dem Körper äußerst schwache Signale zu verarbeiten, die normalerweise im thermischen Rauschen untergehen, und auf technischem Weg nicht erfasst werden können. Es ist einleuchtend, dass die hohe Empfindlichkeit biologischer Systeme nur durch ein, im Vergleich zu technischen Lösungen, hohes Signal/Rausch-Verhältnis erreicht werden kann. Um die extrem hohe Sensitivität zu erzielen, wie wir sie in biologischen Systemen vorfinden, müssen diese in der Lage sein, einlaufende Signale vom Untergrundrauschen zu trennen, und verstärken zu können. Für chaotische Impulse hingegen muss es weitgehend durchlässig sein, während es andererseits in der Lage sein muss Signale herauszufiltern.

Ein derart hohes Signal/Rausch-Verhältnis spricht einerseits für eine extrem hohe Kohärenz, andererseits für die enorme Anpassungsfähigkeit des biologischen Organismus, welche thermodynamisch betrachtet nur zu erreichen ist, durch einen Phasenübergang zwischen einem chaotischen und einem geordnetem System (Prigogine). Das heißt, das System muss flexibel reagieren können um Signale optimal nutzen, bzw. vermeiden zu können. Das biologische System arbeitet deshalb an der Laserschwelle, durch welche garantiert wird, dass bereits Impulse von minimaler Intensität zu Veränderungen führen, und damit potentiell zur sinnvollen Strukturierung des Systems führen können. Bei Krankheit ist die Kohärenz der vom Gewebe emittierten Strahlung nachweislich reduziert, womit auf physikalischer Ebene Krankheit als Kohärenzverlust verstanden werden kann. Ziel einer phamakologischen Therapie sollte somit sein, einerseits die Resonanz zu energetisch gestörten Bereichen herzustellen und gleichzeitig die Kohärenz zu verbessern. Die vorliegende Erfindung zeigt verschiedene Mechanismen sowie aus diesen abgeleitete Präparate bzw. Präparate-Systeme auf, diese Ziele in optimaler Weise zu erreichen. Dies gelingt im Einzelnen durch die Verwendung einer polaren Trägersubstanz und/oder eines Multiresonators in Verbindung mit einem oder mehreren Wirkstoffen. Die Bedeutung und Wirkungsweise soll im Folgenden kurz dargestellt werden:

### Polarität

Energetische Tests in Form von Elektroakupunktur-Diagnose zeigen, dass oftmals bereits allein die Gabe der pathologisch reduzierten Polarität in der Lage ist, zumindest bis zu einem gewissen Grad, eine Normalisierung des Resonanzverhaltens im betreffenden Gewebebezirk zu erreichen. Dies deutet darauf hin, dass hierdurch der physiologische Rhythmus des vorher gestörten Gewebes positiv beeinflusst wird, und damit auch die Resonanzeigenschaften des Gewebes verbessert werden.

Hieraus kann man folgern, dass energetische Defizite oder Krankheit immer auch mit einer Schwächung der Polarität der betroffenen Gewebe verbunden sind. Dies trifft, wie Tests zeigen, im Wesentlichen auch auf die polaren Eigenschaften von Präparaten zu. Dabei zeigt sich, dass in Abhängigkeit von Krankheitsgeschehen und verwendetem Wirkstoff, sowie dessen Konzentration, sowohl mittels Plus- oder Minus-Polarität häufig eine Verbesserung der pharmakologischen Wirkung erreicht werden kann. Der Wirkungsmechanismus beruht darauf, dass durch Normalisierung der gestörten Polarität, die Wiederherstellung der physiologischen Oszillationen des gestörten Gewebebezirks begünstigt werden, und damit auch die Kohärenz erhöht wird. So zeigen beispielsweise energetischeTests (EAV), dass es gelingt durch die therapeutische Applikation von polaren/polarisierten Schwingungen, die Kohärenz, und damit die Gewebefunktionen oft signifikant zu verbessern. Man erkennt hieran, dass dieser Effekt nicht primär vom Wirkstoff abhängt.

Der beschriebene Effekt wirkt sich zusätzlich günstig auf die Signalverarbeitung aus, d.h. das Schwingungsmuster des Arzneimittels kann so vom Organismus besser erkannt und verarbeitet werden. Das Problem besteht nämlich darin, dass kranke, und damit energetisch gestörte Gewebebezirke ganz allgemein in geringerem Maße mit Schwingungen und damit auch mit Präparaten aller Art in Resonanz gehen. Dies ist der Grund dafür, dass oftmals Präparate, beispielsweise Medikamente oder Nahrungsergänzungsmitteln, nicht die gewünschten Wirkungen entfalten.

Dies legt die Schlussfolgerung nahe, dass das in seiner physiologischen Polarität beeinträchtigte Gewebe an zeitlich-räumlicher Strukturierung durch die damit veränderte rhythmische Aktivität einbüßt. Von einer Schwächung der Polarität ist vor allem auch die Wirksamkeit der Enzyme betroffen. Dabei spielt hier nicht nur die pH-Abhängigkeit der Enzymwirkungen eine Rolle, sondern auch die zeitliche Abstimmung der Enzymaktivitäten. Es wird angenommen, dass diese an gekoppelte Schwingungsmoden gebunden sind, durch welche bewirkt wird, dass die Enzyme, die nicht gleichmäßig aktiv sind, zum richtigen Zeitpunkt aktiviert oder deaktiviert werden. Diese Rhythmen und Prozesse werden durch Polaritätsstörungen naturgemäß beeinträchtigt. Im Gegensatz zu ultrafeinen polaren Schwingungen haben aber Säuren und Basen, aber auch viel Wirkstoffe und Zubereitungen auch ein gewisses toxisches Potential, insbesondere auf bereits geschädigte Gewebe, welches hierfür naturgemäß besonders anfällig ist. In vielen Fällen sind deshalb hohe Verdünnungen oder homöopathische Aufbereitungen der polaren Trägersubstanz sinnvoll.

Von Interesse in diesem Zusammenhang sind Versuche die zeigen, dass die optimale Wirkung der homöopathischen Verdünnung (Potenzierung) an die Anwesenheit eines polaren Lösungsmittels gebunden ist. Üblicherweise wird eine Wasser-Alkohol-Mischung verwendet, wobei Wasser wie auch Alkohol, polare Flüssigkeiten darstellen, und deshalb als Lösungsmittel für homöopathische Präparate geeignet sind.

Wesentlich für die Erfindung ist nun die Erkenntnis, dass nicht primär die Verdünnung, sondern die mit der Verdünnung verbundene, relativ stärkere Polarisierung infolge der Dipoleigenschaften des Wassers, der entscheidende Faktor bei homöopathischen Präparaten ist. Da der polare Charakter durch das Lösungsmittel geprägt wird, tritt bei höheren Verdünnungen im Vergleich zur Urtinktur der polare Charakter stärker hervor, und die Resonanzeigenschaften des Präparats werden tendenziell verbessert. Ausgehend von dieser Erkenntnis kann man die Resonanzeigenschaften von Präparaten aller Art durch Polarisierung (pH-Wert / Redox-Potential) erheblich verbessern. Dabei bleibt die positive Wirkung auf die Resonanzeigenschaften eines Präparates auch bei sehr hohen Verdünnungen, beispielsweise im Bereich einer D15 oder D20 deutlich erkennbar erhalten, wodurch die erfindungsgemäßen Zubereitungen ebenso hervorragend zu Optimierung und Herstellung von homöopathischen Präparaten geeignet sind.

Kennzeichnend für eine bevorzugte Ausführungsform der erfindungsgemäßen Präparate ist somit, dass die Präparate monopolaren, also plus- oder minuspolaren Charakter aufweisen, also entweder sauer oder basisch sind, wobei bei höher verdünnten, insbesondere homöopathischen Präparaten zumindest die Ausgangslösungen (sog Urtinkturen) entweder sauer oder basisch eingestellt sind. Speziell bei homöopathischen Verdünnungen, sog. Potenzen, kommt es insbesondere bei mittleren oder höheren Potenzen zu einer starken Abschwächung des sauren oder basischen Charakters, so dass diese im fertigen Produkt nicht mehr gemessen werden können. Gleichwohl ist auch ein extrem schwacher, monopolarer, also Minus- oder Plus Charakter, für die speziellen Eigenschaften der erfindungsgemäßen homöopathischen Zubereitungen hinsichtlich der Wirkung von entscheidender Bedeutung.

### Breitbandresonator

Die zweite Komponente des erfindungsgemäßen Produktes stellt der Breitband- oder Multiresonator dar. Mittels diesem können die Kohärenz- und Resonanzeigenschaften von Präparaten in vielen Fällen noch weiter verbessert werden. Geht man vom technischen Modell aus, ist dies insofern überraschend, weil die Bedingungen für eine hohe Resonatorgüte, wie sie in der Technik bestehen, nicht gegeben sind.

Für einen technischen Empfänger gilt nämlich, dass das Signal/Rausch-Verhältnis bei tiefen Temperaturen und schmalem Frequenzband günstiger wird. Von biologischen Systemen aber weiß man, dass diese im Gegensatz zu technischen Systemen nicht in niedrigen Temperaturbereichen arbeiten und ebenso wenig nur in einem schmalen Frequenzbereich kommunizieren.

Während also das technische System zur Leistungssteigerung im Sinne einer Erhöhung des Signal-Rausch-Verhältnisses auf eine Verringerung der Störungen und einer Einschränkung des Kommunikationsbereiches, und damit der Komplexität und des Informationsgehaltes angewiesen sind, sind biologische Systeme auch bei Störungen, wie sie durch das thermisches Rauschen bei höheren Temperatur (z.B. 37° Körpertemperatur beim Menschen) zwangsläufig entstehen hierzu durchaus in der Lage.

Um diese Leistung zu erbringen ist eine hohe Kohärenz (Ordnung) des biologischen Systems erforderlich. Wie das Phänomen der Biolumineszenz (F.Popp) nahe legt, verfügen biologische Systeme über eine ungleich höhere Kohärenz als der beste technisch herstellbare Laser, und damit auch über optimale Voraussetzungen zur Signalverarbeitung. Bei Krankheit oder Funktionsstörung ist die Kohärenz nachweislich verringert. Dies bedeutet, dass damit auch die Signalverarbeitung gestört ist, und deshalb Stoffe aller Art auch schlechter verarbeitet werden können.

Ausgehend vom Schwingungsmodell werden, wie bereits erläutert, die Wirkungen von Stoffen, in unserem Fall die von Arzneimitteln oder Nahrungsergänzungsmitteln als Resonanzeffekte gedeutet. Arzneimittel werden in diesem Modell somit als (Sender) aufgefasst und der Körper als Empfänger. Die Aufgabe von Arzneimitteln besteht entsprechend dem Modell darin, dem Körper dazu zu verhelfen, ein krankhaft verändertes Schwingungsmuster im wieder in die ursprüngliche Ordnung zurückzuführen.

Dieser Negativ-Effekt kann jedoch überraschenderweise beseitigt und sogar überkompensiert werden, wenn zusätzlich zur Polarisierung dem Präparat Resonatoren vorzugsweise in Form von Mineralstoffen und Spurenelementen zugefügt werden. Hierzu reichen erstaunlicherweise vergleichsweise geringe Konzentrationen aus. Diese haben die Funktion eines BreitbandResonators. Dieser positive Effekt ist insofern nicht zu erwarten, da, wie in diesem Zusammenhang angesprochen, eine Verbreiterung des Resonanzspektrums in technischen Systemen zu einer Verschlechterung des Signal-Rausch-Verhältnisses führt. Für das biologische System gelten offenbar andere Gesetzmäßigkeiten als für das technische System.

Ein Verständnis dieser Tatsache wird erst möglich, wenn man den grundlegenden Unterschied berücksichtigt, dass biologische Systeme unter Verbrauch von Energie ihre Ordnung aktiv aufrechterhalten, also thermodynamisch betrachtet, fernab vom thermodynamischen Gleichgewicht arbeiten. Der Körper kämpft, wenn man so will, permanent gegen ein drohendes Chaos an.

Da somit Energie erforderlich ist, um das zur Ordnungsbildung erforderliche thermodynamische Ungleichgewicht aufrecht zu erhalten, kann bei Energiemangel der Körper seine Ordnung nur noch in reduziertem Maße bewahren. Hier setzt die Wirkung des erfindungsgemäßen Breitbandresonators an. Dieser ist in der Lage, das zeigen die energetischen Tests, über nichtlokale Effekte die Kohärenz im Organismus zu verbessern. Da eigentlich Mineralstoffe als Resonatoren das Signal-Rausch Verhältnis verschlechtern müssten, ist davon auszugehen, dass dieser Negativ-Effekt über andere Mechanismen überkompensiert wird.

Zunächst ist noch die Tatsache zu beachten, dass die günstige Wirkung der Breitbandresonatoren und insbesondere von Mineralstoffen im Sinne eine Verbesserung der Kohärenz sowohl von der Konzentration, wie besonders auch von dem Polarisationsgrad, sowie der richtigen Polarität abhängig ist.

Die Erfahrung aus den Bioresonanz-Tests zeigt weiter, dass durch geeignete Resonatoren eine signifikante Steigerung des Energielevels der angesprochenen Frequenzbereiche zu erreichen ist. Da die meisten Enzyme Mineralien enthalten, kann man sich vorstellen, dass insbesondere die Mineralstoff-Resonatoren eine Steigerung der Enzym-Aktivität bewirken, und so über die Anhebung des Energie-Levels eine Erhöhung der Kohärenz des biologischen Feldes ermöglichen. Dieser Effekt dürfte vor allem da überproportional zutrage treten, wo vorher, beispielsweise durch enzymatische Funktionsdefizite eine ungünstige Energiesituation besteht.

Dies bedeutet weiter, dass damit die Signalverarbeitung gerade im gestörten oder kranken Gewebebezirk gestört ist. Diese wirkt sich in vielerlei Hinsicht nachteilig aus, und behindert folglich auch die Resorption und Bioverfügbarkeit von Stoffen aller Art, sowie die Wirksamkeit von Nahrungsergänzungsmitteln, kosmetischen Wirkstoffen und Arzneimitteln etc. insbesondere dort, wo diese ihre Wirkung entfalten sollen.

Die Nachteile sind im Einzelnen:
- Reduktion des internen Informationsflusses
- Energetische Abkopplung des kranken Gewebebezirkes
- Reduzierte Trennschärfe bei der Signalverarbeitung. Dadurch verminderte organotrope Wirkung.
- Schlechtere Mustererkennung: Gefahr immunologischer Verwechslungen erhöht > erhöhte Gefahr von Autoimmunprozessen, allergischen Reaktionen (z. B auch auf Arzneimittel)
- Ganzheitliche Entfaltung der Wirkung wie sie beim Einsatz insbesondere von Phytotherapeutica und Homöopathika angestrebt wird, ist deutlich reduziert. Damit wird das Präparat vom Organismus verfälscht wahrgenommen.

Dabei ist zu berücksichtigen, dass der Stoffwechsel selbst auf die hohe Kohärenz angewiesen ist, und dass nur durch diese die hohen Stoffwechselraten, wie sie in biologischen Systemen anzutreffen sind, erreicht werden können.

Ausgehend von diesen Überlegungen hat der Erfinder überraschend gefunden, dass durch eine polare Trägersubstanz, insbesondere in monopolarer Form, vorzugsweise auf der Basis von Säuren oder Basen oder durch eine Kombination von einer oder mehreren polaren Trägersubstanzen mit einem oder mehreren Breitbandresonatoren die Möglichkeit gegeben ist, Wirkstoffen die erforderliche Resonanz im biologischen System zu verschaffen, so dass diese optimal wirken können. Hierbei kommt es vorzugsweise auf das Zusammenwirken von polarer Trägersubstanz und Breitbandresonator an, da der Breitbandresonator an sich durch Bereitstellung eines breiten Spektrums an Resonanzen grundsätzlich dämpfend wirkt, anstatt selektiv durch Resonanz einzelne Wirkungen zu verstärken. Dies hat aber zugleich den positiven Effekt, dass Nebenwirkungen abgeschwächt oder vermieden werden können. Dazu ist es erforderlich, eine Abstimmung von polarer Trägersubstanz und Breitbandresonator zu treffen, also das Verhältnis von Polarität zur Konzentration des Breitbandresonators und/oder die Konzentration des Breitband- oder Multiresonators einzustellen. Insbesondere dient das Präparat zur Vergrößerung der therapeutischen Breite, d. h. bei Steigerung der Wirkung im Sinne der arzneilichen Wirksamkeit werden gleichzeitig die unerwünschten Wirkungen (sog. Nebenwirkungen) reduziert. Die eine bevorzugte Ausführungsform des erfindungsgemäßen Präparats mit polarer Trägersubstanz und Breitbandresonator weist dabei die überraschende Eigenschaft auf, dass mittels diesem auch bei identischer Konzentration des oder der Wirkstoffe, gleichzeitig die Wirksamkeit verbessert und die Nebenwirkungen reduziert werden können. Der Mediziner spricht hier von Vergrößerung der therapeutischen Breite. In der Praxis ergibt sich somit zusätzlich die die Möglichkeit der Dosisverringerung, bei gleich bleibender oder sogar besserer Wirksamkeit des Präparates.

Vorzugsweise wird bei toxischen Wirkstoffen von nicht homöopathischer Art eine höhere Resonatorenkonzentration zur Dämpfung der toxischen Nebenwirkung eingesetzt, während bei homöopathischen Wirkstoffen die Resonatorenkonzentration kleiner gewählt wird, je toxischer der Wirkstoff ist.

Üblicherweise liegt die Resonatorenkonzentration im Bereich der Wirkstoffkonzentration oder wenige Dezimalpotenzen darüber oder darunter, wobei unter Potenzen als Grad der homöopathischen Verdünnung zu verstehen ist.

Je höher die Resonatorenkonzentration ist, desto stärker wird vorzugsweise die Polarität eingestellt, um die Übertragung der gewünschten Wirkung auf das biologische System sicher zu stellen.

Um die Empfänglichkeit des biologischen Systems zu berücksichtigen, kann es vorteilhaft sein in Abhängigkeit vom Krankheitsbild bzw. dem Verlauf der Therapie unterschiedliche Präparate mit unterschiedlichen Konzentrationen des Breitbandresonators und/oder des Verhältnisses von Polarität zur Resonatorkonzentration und/oder der Stärke der Polarität in Form einer Mehrstufentherapie einzusetzen, wobei entsprechende Mehrkomponentenpräparate Gegenstand der vorliegenden Erfindung sind.

Die Breitbandresonatoren können aus einer Gruppe ausgewählt werden, die Mineralien, Spurenelemente, Emulgatoren, Gelbildner, essentielle oder nichtessentielle Metalle/Metallsalze und/oder Nichtmetalle, insbesondere Quarzsand, Gesteinsmehl, Mineralmehl, natürliche Salze, vorzugsweise Meersalz, Steinsalz oder Himalajasalz, Muschelkalk, Heilerde, Siliziumverbindungen, Methylcellulose, Alginate, Agar-Agar, Johannisbrotkernmehl, Guakernmehl Eisen, Zink, Kupfer, Mangan, Chrom, Molybdän, Vanadium, Nickel, Zinn, Silber, Gold, Jod, Arsen, Silizium, Calcium, Magnesium, Kalium, Natrium, Germanium umfasst. Die Resonatoreneigenschaft für bestimmte Wirkstoffe und/oder einzelne Patienten wird mittels Energetisierungsverfahren, insbesondere gemäß dem Bioresonanz- und/oder Magnetisierungsverfahren festgestellt.

Ein erfindungsgemäßes Präparat kann unmittelbar vor der Anwendung mit dem Wirkstoff vermischt werden oder in zeitlicher Nähe zusammen mit dem Wirkstoff eingenommen werden, oder es kann als Fertigpräparat einen oder mehrere Wirkstoffe enthalten.

Im Falle dass die Anwesenheit zur Störung der energetisch-physikalischen Eigenschaften des Präparats führt, kann auf den Einsatz von Metallen oder Verbindungen, die Eisen, Kupfer oder Mangan enthalten, verzichtet werden.

Da die Breitbandresonatoren von Hause aus durch einen großen Resonanzbereich dämpfende Eigenschaften aufweisen, kann bei einem Präparat mit oder ohne polare Trägersubstanz, bei der also ein Teil der Kombination zur Übertragung der positiven Wirkung auf das biologische System fehlt, die negative Wirkung von Stoffen, wie Allergenen oder dgl. gedämpft oder beseitigt werden. Das Ziel ist eine selektive Dämpfung ungünstiger Schwingungen.

Nach dem der vorliegenden Erfindung zu Grunde liegenden Modell beruhen sämtliche biologischen, chemischen und pharmakologischen Wirkungen letztlich auf Schwingungen/Wellen und deren Wechselwirkungen und deshalb besitzen zur Optimierung von Präparaten oder Formulierungen die physikalischen gleichermaßen wie die chemischen Eigenschaften die entscheidende Bedeutung. Sie schließen quasi mechanische Vorstellungen der Enzymtheorie wie Schlüssel-Schloss-Prinzip oder der des Rezeptors in der Pharmakologie mit ein, die als Grenzfall des Wellenmodells aufgefasst werden können. Da es sich hier um ein grundlegendes und universelles Prinzip handelt besitzt dieses Gültigkeit für alle Formen der Wirksamkeit von Stoffen auf den Organismus. Dabei sind die Übergänge von der Physik zur Chemie naturgemäß fließend, so dass die die Erfindung kennzeichnenden Parameter sowohl mit physikalischen wie chemischen Begriffen beschrieben werden können.

Ausgehend vom Wellenmodell bedarf es zur Erzielung einer Wirkung der Resonanz. Mittels Resonanzwirkungen ist eine selektive Übertragung von Wirkungen möglich. Dies schließt sowohl lokale Effekte wie auch nicht nicht-lokale Wirkungen mit ein. Dabei lässt sich der biologische Organismus als ein Detektorsystem auffassen, das bei der Verarbeitung von Reizen vor die Aufgabe gestellt ist, informative Schwingungen (Signale) von Schwingungen ohne Informationen (Thermisches Rauschen) zu trennen. Dabei gelingt es dem Körper äußerst schwache Signale zu verarbeiten, die normalerweise im thermischen Rauschen untergehen, und auf technischem Weg nicht erfasst werden können. Es ist evident, dass die hohe Empfindlichkeit biologischer Systeme nur durch ein, im Vergleich zu technischen Lösungen, extrem hohes Signal/Rausch -Verhältnisses erreicht werden kann. Dabei wird das biologische System als Detektor aufgefasst. Um eine extrem hohe Sensitivität zu erzielen, wie wir sie in biologischen Systemen vorfinden, müssen diese in der Lage sein, einlaufende Signale vom Untergrundrauschen zu trennen, und verstärken zu können. Für chaotische Impulse hingegen muss es weitgehend durchlässig sein, während es andererseits in der Lage sein muss echte Signale herauszufiltern.

Ein derart hohes Signal/Rausch-Verhältnis ist thermodynamisch betrachtet nur zu erreichen, durch einen Phasenübergang zwischen einem chaotischen und einem geordnetem System (Prigogine). Das heißt, das System muss flexibel reagieren können um Signale optimal nutzen, bzw. vermeiden zu können. Das biologische System arbeitet deshalb an der Laserschwelle, durch welche garantiert wird, dass bereits Impulse von minimaler Intensität zu Veränderungen führen, und damit potentiell zur Strukturierung führen können.

Diese besonderen Eigenschaften von biologischen Systemen die dazu beitragen, dass Informationen extrem effektiv verarbeitet werden können, sind, geht man vom technischen Modell aus, zunächst überraschend, weil die Bedingung für eine hohe Resonatorgüte, wie sie in der Technik bestehen nicht gegeben sind. Für einen technischen Empfänger gilt nämlich, dass das Signal/Rausch-Verhältnis bei tiefen Temperaturen und schmalem Frequenzband günstiger wird. Von biologischen Systemen aber weiß man, dass diese im Gegensatz zu technischen Systemen nicht in niedrigen Temperaturbereichen arbeiten und ebenso wenig nur in einem schmalen Frequenzbereich kommunizieren.

Während also das technische System zur Leistungssteigerung im Sinne einer Erhöhung des Signal-Rausch-Verhältnisses auf eine Verringerung der Störungen und einer Einschränkung des Kommunikationsbereiches, und damit der Komplexität und des Informationsgehaltes angewiesen sind, sind biologische Systeme auch bei Störungen, wie sie durch das thermisches Rauschen bei höheren Temperatur(z.B. 37°Körpertemperatur beim Menschen) zwangsläufig entstehen hierzu durchaus in der Lage.

Um diese Leistung zu erbringen ist eine hohe Kohärenz (Ordnung) des biologischen Systems erforderlich. Wie das Phänomen der Biolumineszenz (F.Popp) nahe legt, verfügen biologische Systeme über eine ungleich höhere Kohärenz als der beste technisch herstellbare Laser, und damit auch über optimale Voraussetzungen zur Signalverarbeitung. Bei Krankheit oder Funktionsstörung ist die Kohärenz nachweislich verringert. Dies bedeutet, dass damit auch die Signalverarbeitung gestört ist, und deshalb Stoffe aller Art auch schlechter verarbeitet werden können.

Dies bedeutet, dass damit die Signalverarbeitung gerade im gestörten oder kranken Gewebebezirk gestört ist. Diese wirkt sich in vielerlei Hinsicht nachteilig aus, und behindert folglich auch die Resorption und Bioverfügbarkeit von Stoffen aller Art, sowie die Wirksamkeit von Nahrungsergänzungsmitteln, kosmetischen Wirkstoffen und Arzneimitteln etc. insbesondere dort, wo diese ihre Wirkung entfalten sollen. Die Nachteile sind im Einzelnen:
- Reduktion des internen Informationsflusses
- Energetische Abkopplung des kranken Gewebebezirkes
- Reduzierte Trennschärfe bei der Signalverarbeitung. Dadurch verminderte organotrope Wirkung.
- Schlechtere Mustererkennung: Gefahr immunologischer Verwechslungen erhöht > erhöhte Gefahr von Autoimmunprozessen, allergischen Reaktionen (z. B auch auf Arzneimittel)
- Ganzheitliche Entfaltung der Wirkung wie sie beim Einsatz insbesondere von Phytotherapeutica und Homöopathika angestrebt wird, ist deutlich reduziert. Damit wird das Präparat vom Organismus verfälscht wahrgenommen.

Dabei ist zu berücksichtigen, dass der Stoffwechsel selbst auf die hohe Kohärenz angewiesen ist, und dass nur durch diese die hohen Stoffwechselraten, wie sie in biologischen Systemen anzutreffen sind, erreicht werden können.

Energetische Tests in Form von Elektroakupunktur-Diagnose lassen nun den Schluss zu, dass eine Abnahme der Kohärenz immer auch mit einer Depolarisation der betroffenen Gewebe verbunden ist. und umgekehrt durch die Applikation von polaren Schwingungen die Kohärenz und damit die normale Signalverarbeitung wieder hergestellt werden kann. Weiter zeigt sich, dass es gelingt durch die therapeutische Applikation von polaren/polarisierten Schwingungen, die Kohärenz, und damit die Gewebefunktionen praktisch immer signifikant zu verbessern.

Ausgehend vom Schwingungsmodell werden, wie bereits erläutert, die Wirkungen von Stoffen, in unserem Fall die von Arzneimitteln als Resonanzeffekte gedeutet. Arzneimittel werden in diesem Modell somit als (Sender) aufgefasst und der Körper als Empfänger. Die Aufgabe von Arzneimitteln besteht entsprechend dem Modell darin, dem Körper dazu zu verhelfen, ein krankhaft verändertes Schwingungsmuster im wieder in die ursprüngliche Ordnung zurückzuführen.

Diese Vorstellungen vorausgesetzt, kann man Erfahrungen mit der Wirkung von Schwingungen auf den Körper aus anderen Bereichen heranziehen. Von besonderem Interesse sind hierbei die Forschungen im Zusammenhang mit sog. ELF-sferics (Extreme Low Frequency), dessen bekanntestes Beispiel die sog. Schumannwellen (atmosphärische Wellen) darstellen.

Diese entstehen in einem riesigen Kugelschalen-Resonator der einerseits durch die Ionosspäre, genauer gesagt deren Unterseite (sog. Heavy-side-Schicht) und der Erdoberfäche begrenzt wird. Die Frequenz liegt bei 7,8 Hz. Die ist insofern von Interesse, da, 7,8 Hz genau die Frequenz ist, die bei allen Säugern genau übereinstimmen. Forschungen des Max-Planck-Instituts (im Auftrag der NASA) ergaben, dass der Mensch Schumannwellen als biotrope Reize benötigt. Aus diesem Grund baut die NASA in ihre bemannten Satelliten Schumannwellen-Generatoren ein.

Man hat nun festgestellt, dass die künstlich Zufuhr von Schumannwellen durch einen elektromagnetischen Frequenzgenerator bei den Testpersonen sehr oft zu einer raschen Normalisierung der zirkadianen Rhythmen führt und die Wetterfühligkeit reduzieren kann. Gleichzeitig beobachtete man, dass bei längerer Applikation von Schumannwellen Thrombozyten-Adhäsivitätsstörungen bis in pathologische Bereiche auftreten. Diese, als Nebenwirkungen einzustufenden Veränderungen, verschwanden, wenn zusätzlich die Elektronenplasma-Wellen der 30 wichtigsten Spurenelemente hinzu gegeben wurden.

Aus diesen Beobachtungen kann man nun die ganz allgemeine Folgerung ziehen, dass eine Erweiterung des Spektrums die Schädlichkeit der Strahlung vermindert. Von Interesse ist, dass damit nicht nur die Nebenwirkung der Schumann-Strahlung zu 100% beseitigt wird, sondern sich gleichzeitig auch die Wirkung auf das Symptom Wetterfühligkeit von 65% auf 95% steigern lässt.

Fasst man also sämtliche Erscheinungen als Materie-Schwingungen auf, dann lassen sich diese Erkenntnisse verallgemeinern und auch auf die Anwendung von stofflichen Zusammensetzungen nutzen, um entsprechend Präparate mit Breitbrandresonator formulieren.

Dieser Negativ-Effekt kann jedoch überraschenderweise beseitigt und sogar überkompensiert werden, wenn zusätzlich zur Polarisierung dem Präparat Resonatoren vorzugsweise in Form von Mineralstoffen und Spurenelementen zugefügt werden. Hierzu reichen erstaunlicherweise vergleichsweise geringe Konzentrationen aus. Diese haben die Funktion eines BreitbandResonators. Dieser positive Effekt ist insofern nicht zu erwarten, da, wie in diesem Zusammenhang angesprochen, eine Verbreiterung des Resonanzspektrums in technischen Systemen zu einer Verschlechterung des Signal-Rausch-Verhältnisses führt. Für das biologische System gelten offenbar andere Gesetzmäßigkeiten als für das technische System.

Ein Verständnis dieser Tatsache wird erst möglich, wenn man den grundlegenden Unterschied berücksichtigt, dass biologische Systeme unter Verbrauch von Energie ihre Ordnung aktiv aufrechterhalten, also thermodynamisch betrachtet, fernab vom thermodynamischen Gleichgewicht arbeiten. Der Körper arbeitet, wenn man so will, permanent gegen ein drohendes Chaos an.

Da somit Energie erforderlich ist, um das zur Ordnungsbildung erforderliche thermodynamische Ungleichgewicht aufrecht zu erhalten, kann bei Energiemangel der Körper seine Ordnung nur noch in reduziertem Maße bewahren. Hier setzt die Wirkung des erfindungsgemäßen Breitbandresonators an. Dieser ist in der Lage, das zeigen die energetischen Tests, über nichtlokale Effekte die Kohärenz im Organismus zu verbessern. Da eigentlich Mineralstoffe als Resonatoren das Signal-Rausch Verhältnis verschlechtern müssten, ist davon auszugehen, dass dieser Negativ-Effekt über andere Mechanismen überkompensiert wird. Zunächst ist noch die Tatsache zu beachten, dass die günstige Wirkung der Breitbandresonatoren und insbesondere von Mineralstoffen im Sinne eine Verbesserung der Kohärenz sowohl von der Konzentration, wie besonders auch von dem Polarisationsgrad, sowie der richtigen Polarität abhängig ist.

Die Erfahrung aus den Bioresonanz-Tests zeigt weiter, dass durch geeignete Resonatoren eine signifikante Steigerung des Energielevels der angesprochenen Frequenzbereiche zu erreichen ist. Da die meisten Enzyme Mineralien enthalten, kann man sich vorstellen, dass insbesondere die Mineralstoff-Resonatoren eine Steigerung der Enzym-Aktivität bewirken, und so über die Anhebung des Energie-Levels eine Erhöhung der Kohärenz des biologischen Feldes ermöglichen. Dieser Effekt dürfte vor allem da überproportional zutrage treten, wo vorher, beispielsweise durch enzymatische Funktionsdefizite eine ungünstige Energiesituation besteht.

So ist es weiter vorstellbar, dass die Applikation der fehlenden Polarität, den gesunden Rhythmus des vorher gestörten Gewebes wiederherstellt. Es ist nämlich davon auszugehen, dass das in seiner natürlichen Polarität beeinträchtigte Gewebe an zeitlich-räumlicher Strukturierung durch die damit veränderte rhythmische Aktivität einbüßt. Hiervon ist natürlich auch die Wirksamkeit der Enzyme betroffen. Dabei spielt hier nicht nur die pH-Abhängigkeit der Enzymwirkungen eine Rolle, sondern auch die zeitliche Abstimmung der Enzymaktivitäten. Diese sind, an gekoppelte Schwingungsmoden gebunden, durch welche bewirkt wird, dass die Enzyme, die nicht gleichmäßig aktiv sind, zum richtigen Zeitpunkt aktiviert oder deaktiviert werden. Diese Rhythmen und Prozesse werden durch Polaritätsstörungen naturgemäß beeinträchtigt. Durch Zugabe der geschwächten Polarität, lässt sich tendenziell eine Normalisierung des Rhythmus erreichen. Im Gegensatz zu ultrafeinen polaren Schwingungen haben aber Säuren und Basen, aber auch viel Wirkstoffe und Zubereitungen ein nicht unerhebliches toxisches Potential. Das bereits geschädigte Gewebe ist hierfür naturgemäß besonders anfällig, so dass bei stärkerer Störung oder Schädigung die unerwünschten Wirkungen tendenziell überproportional hervortreten.

Das Problem ist also, dass kranke, und damit energetisch gestörte Gewebebezirke in geringerem Maße mit Schwingungen und damit auch mit Nahrungsergänzungsmitteln/Arzneimitteln in Resonanz gehen. Dies ist der Grund dafür, dass oftmals Präparate nicht die die gewünschten Wirkungen entfalten. Die Wirkung der homöopathischen Verdünnung (Potenzierung) ist an die Anwesenheit eines polaren Lösungsmittels gebunden. Üblicherweise wird eine Wasser-Alkohol-Mischung verwendet, wobei Wasser wie auch Alkohol, polare Flüssigkeiten darstellen, und deshalb als Lösungsmittel für homöopathische Präparate geeignet sind. Allerdings kann auch Zucker als polare Substanz angesehen werden. Wesentlich für die Erfindung ist nun die Erkenntnis, dass nicht primär die Verdünnung, sondern die mit der Verdünnung verbundene relativ stärkere Polarisierung der entscheidende Faktor ist. Da der polare Charakter durch das Lösungsmittel geprägt wird, tritt bei höheren Verdünnungen im Vergleich zur Urtinktur der polare Charakter stärker hervor, und die Resonanzeigenschaften des Präparats werden tendenziell verbessert. Ausgehend von dieser Erkenntnis kann man die Resonanzeigenschaften von Präparaten aller Art durch Polarisierung (pH-Wert/Redox-Potential) und gleichzeitigen Einsatz eines Multi-oder Breitbandresonators verbessern. Die erfindungsgemäße Zubereitung enthält demnach vorzugsweise folgende Komponenten:
1. Multiresonator
   Metalle und Spurenelemente können als Resonatoren eingesetzt werden, da sie über ihre spezifischen Eigenschwingungen als Bestandteil von Enzymen, Vitaminen und Hormonen deren Eigenschaften entscheidend beeinflussen. In Teilen oder in der Gesamtheit bilden sie Multiresonatoren, die für die Fähigkeit der Organismus zur Verarbeitung von Schwingungen, und damit für die Steuerung des Stoffwechsels von grundlegender Bedeutung sind. Die erfindungsgemäße Zubereitung enthält vorzugsweise einen solchen Multiresonator oder Breitbandresonator in Form von Metallen (Eisen, Kupfer.Zink usw.) in kolloidaler Form oder von Metallsalzen in Lösung, und /oder nichtmetallischen Spurenelementen. Bevorzugt wird hierzu ein breiteres Spektrum von Metallen und Spurenelementen verwendet. Ferner können auch natürliche Zusammensetzungen wie z. B. Kochsalz, Steinsalz, Himalajasalz (breites Spurenelement-Spektrum) oder Muschelkalk verwendet werden. Diese werden im Vergleich zur Supplementierung nur in geringer Menge eingesetzt. Zweck des Resonators ist eben nicht die Supplementierung, sondern die Nutzung der Multiresonator-Eigenschaften. Voraussetzung hierfür ist jedoch, dass diese hinschtlich der Polarität den Bedürfnissen des Organismus bzw. der angesteuerten Gewebe angepasst sind.
2. Polares System
   Kennzeichnend ist weiterhin, dass die Präparate polaren Charakter aufweisen, also entweder sauer oder basisch und/oder ein entsprechendes Redoxpotential aufweisen, wobei insbesondere bei homöopathischen Präparaten zumindest die Ausgangslösungen entsprechend eingestellt sind. Bei homöopathischen Verdünnungen, sog. Potenzen, kommt es insbesondere bei mittleren oder höheren Potenzen zu einer starken Abschwächung des sauren oder basischen Charakters und somit im fertigen Produkt event. nicht mehr gemessen werden können. Gleichwohl ist der, durch die Formulierung der Ausgangslösung erreichte, wenn auch schwache polare Charakter, für die speziellen Eigenschaften der homöopathischen Zubereitungen von Bedeutung. Für homöopathische Zubereitungen ist demnach das homöopathische Verfahren mit den einzelnen Herstellungsschritten zu berücksichtigen.
3. Wirkstoff-Komponente
   Der polare Resonator stellt eine elektrochemische Gleitschiene dar, die im engerem Sinne nicht zu den wirksamen Bestandteilen eines Fertigpräparats mit Wirkstoffkomponente gerechnet werden kann. Sie dient lediglich der Optimierung der Resonanz, welche durch eine energetische Aktivierung der Erfolgsgewebe erreicht wird. Durch diesen Effekt gelangen die vorzugsweise im Fertigpräparat enthalten spezifischen Wirkstoffe zu einer besseren Entfaltung. Gleichzeitig werden durch den ausgleichenden Effekt des oder der Multiresonatoren die Nebenwirkungen reduziert. Die energetischen Tests mittels Elektroakkupunktur oder anderen energetischen Testverfahren widerlegen die weit verbreitete Vorstellung, dass eine Verstärkung der Wirkung immer auch mit einer Zunahme unerwünschter Wirkungen, sog. Nebenwirkungen verbunden sein muss.
   Den Funktionsmechanismus kann man sich etwa wie folgt vorstellen: Der Körper hat prinzipiell die Möglichkeit belastende Wellen zu absorbieren (auch Substanzen haben Wellencharakter) oder hindurch laufen zu lassen, ohne dass diese Schaden anrichten. Dies beruht darauf, dass biologische Systeme an der Laserschwelle arbeiten und damit in der Lage sind in hohem Maße die einlaufenden Wellen zu selektieren. Ist die Kohärenz des Gewebes z.B. bei Krankheit reduziert, dann ist auch die Energie- und Signalverarbeitung des Gewebes beeinträchtigt und die gewünschte präzise Selektion von positiv oder negativ wirkenden Schwingungen ist nicht mehr wie beim Gesunden gegeben.
   Energetische Tests zeigen nun, dass Kohärenzverluste immer mit unausgeglichenen Polaritäten, und damit auch mit einem unausgeglichenen Energiesystem im Zusammenhang stehen. Durch die Polaritätsverschiebungen werden die normalen Oszillationen des Körper-Energiefeldes vermindert, was eine Reduzierung der Kohärenz zur Folge hat. Tatsächlich sprechen solche Felder sowohl langsamer, als auch schwächer auf exogene Bewellung an. Die Aspekte Polarität und Kohärenz stehen somit im direkten Zusammenhang und reflektieren gleichzeitig die Vitalität des Gewebes.

Im Einzelnen zeigen die Tests, dass
- Multiresonatoren in Abhängigkeit von Konzentration und Polarität sowohl die Resonatorgüte steigern wie auch reduzieren können.
- Je stärker die energetische Störung, Vorschädigung oder Erkrankung des Gewebes ist, desto kleiner das therapeutische Fenster und desto geringer die Bereitsschaft des Gewebes in Resonanz zu gehen. Die angestrebten arzneilichen Wirkungen sind somit schlechter zu erzielen, die Nebenwirkungen treten stärker hervor.
- Es existiert in Verbindung mit den spezifischen Wirkstoffen ein Fenster, innerhalb welchem sich die therapeutische Breite mittels dieses Systems vergrößern lässt.
- Innerhalb dieses Fensters kann man durch die Wahl des Resonators die dämpfende Eigenschaft oder die spezifische Wirkung hervorheben. Mann kann somit die Reizwirkung entsprechend des therapeutischen Ziels und der individuellen Situation exakt anpassen.
- Der Resonator ist der Wirkstoffkonzentration anzupassen. Tests ergaben die Erfahrung, dass in vielen Fällen die Konzentrationen in Bereichen liegt, die im Bereich der Wirkstoffkonzentration, oder wenige Dezimalpotenzen über oder unter der Wirkstoffpotenz liegt. Beispiel: Wirkstoff D6, passende Resonatoren im Bereich D5-D8, je nach therapeutischer Zielsetzung.
- Je höher die Toxizität des Wirkstoffes, desto relativ höher ist die Konzentration des Resonators zu wählen, im Falle von Homöopathica eine niedrigere Potenz.
- Die Verwendung der geeigneten Polarität vermindert die unerwünschten Dämpfungseigenschaften und verbessert so die Resonanz, und damit auch die Wirksamkeit von Präparaten.

Aus den verschiedenen Wirkrichtungen ergeben sich eine Reihe von Anwendungsmöglichkeiten. Diese Anwendungsfälle führen zu einer Vielzahl möglicher Präparate, die auch per Software entsprechend den Grundregeln der Erfindung zusammenstellbar sind. Im Einzelnen sind dies:
Präparate zur energetischen Optimierung und Reduzierung von Nebenwirkungen von Nahrungsergänzungsmitteln, medizinischen/pharmazeutischen Präparaten, Kosmetika, Phytotherapeutica,Homöopathica sowie als Medium zum Aufschwingen von Informationen im Rahmen der Bioresonanz-Therapie oder als Polarer Resontor / Breitbandresonator zur Optimierung von Nahrungsergänzungsmittel (Lebensmittel) und Arzneimitteln, Präparate und
Präparatesysteme für die (energetische) Diagnostik (sog. Testampullen, Testsätze) wie Sie in der alternativen Medizin bekannt und verbreitet sind.

Bei der Anwendung in Arzneimitteln erreicht man mit der erfindungsgemäßen Zubereitung somit eine wesentlich größere therapeutische Breite, d. h. bei gleichzeitiger Verbesserung der gewünschten therapeutischen Wirkung ergibt sich eine signifikante Verringerung der Nebenwirkungen. Gleichzeitig reduziert sich auch in diesem Fall die Allergenität praktisch aller potentiellen Allergene. Die Anwendung des erfindungsgemäßen Zubereitung ist grundsätzlich nicht auf bestimmte Stoffe oder Stoffklassen beschränkt, da diese auf den grundlegenden, oben dargestellten biophysikalischen Prinzipien beruht, wie sie für alle Arten von Wirkungen von Stoffen auf den Organismus gelten. In der Summe erreicht man folgende Effekte:
- Vergrößerung der therapeutischen Breite
- Erhebliche Verringerung der Toxizität von Stoffen und damit von Nebenwirkungen
- Erhöhung der Gewebekohärenz
- Verbesserung der Signalverarbeitung durch Erhöhung der Trennschärfe
- Verringerung von Einsatzkonzentrationen
- Erhöhung der nichtlokalen Wirkung
- Verbesserte Einschleusung von Wirkstoffen durch elektrochemische Gleitschiene
- Verringerung der Allergenität durch verbesserte Mustererkennung des Körpers
- Erhöhung der Wirksamkeit von Arzneimitteln insbesondere an bereits geschädigtem Erfolgsorganen/-Geweben.
- Verbesserung der organotropen Wirkung infolge erhöhter Trennschärfe
- Stimulation der enzymatischen Aktivitäten und des Immunsystems

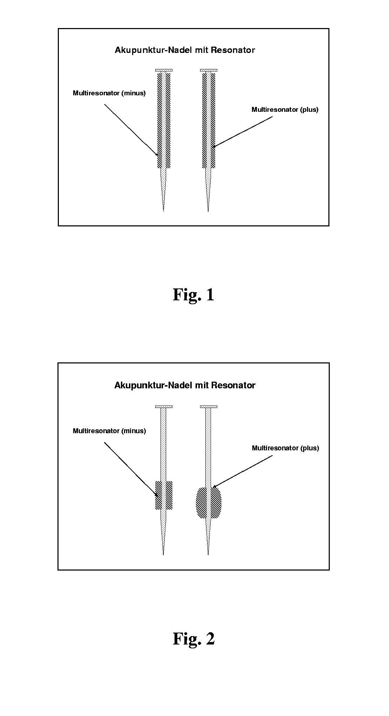

Insbesondere lässt sich die Erfindung somit durch folgende herausragenden Merkmale chrakterisieren:
- Präparat zur Unterstützung der Aufnahme von Wirkstoffen und/ oder Steigerung der Wirksamkeit und/oder der Aufnahme von Nahrungsergänzungsmitteln in einem menschlichen oder tierischen Körper, und/oder zu diagnostischen Zwecken
dadurch gekennzeichnet, dass
mindestens eine polare Trägersubstanz enthalten ist und ein mineralischer, und/oder pflanzlicher, und/oder tierischer und/oder oder synthetischer (chemischer) Wirkstoff enthalten ist.
- Präparat zur Unterstützung der Aufnahme von Wirkstoffen und Steigerung der Wirksamkeit oder der Aufnahme von Nahrungsergänzungsmitteln und Reduzierung von toxischen Wirkungen (Nebenwirkungen) in einem menschlichen oder tierischen Körper, dadurch gekennzeichnet, dass mindestens eine polare Trägersubstanz enthalten ist und ein mineralischer, und/oder pflanzlicher, und/oder tierischer und/oder oder synthetischer (chemischer) Wirkstoff enthalten ist.
- Präparat zur Unterstützung der Aufnahme von arzneilichen Wirkstoffen und/ oder Steigerung der Wirksamkeit oder Aufnahme von Nahrungsergänzungsmitteln und Reduzierung von toxischen Wirkungen(Nebenwirkungen )in einem menschlichen oder tierischen Körper, dadurch gekennzeichnet, dass mindestens eine polare Trägersubstanz und ein Breitbandresonator enthalten ist.
- Präparat, dadurch gekennzeichnet, dass die polare Trägersubstanz eine saure oder basische Lösung oder eine Verdünnung einer derartigen Lösung ist, und aus einem oder mehreren polaren Lösungsmitteln, beispielsweise Wasser oder Alkohol oder einer Mischung derselben besteht, wobei insbesondere die Verdünnung so stark sein kann, dass ein saurer oder basischer Charakter mit gängigen Methoden der pH-Wert-Messung nicht mehr nachweisbar ist.
- Präparat zur Reduzierung toxischer Wirkungen und Verbesserung der Verträglichkeit von Substanzen, die auf den menschlichen oder tierischen Körper einwirken, durch Beimischung, dadurch gekennzeichnet, dass mindestens ein Breitbandresonator enthalten ist.
- Präparat, dadurch gekennzeichnet, dass eines oder mehrere ätherische Öle, insbesondere auch Parfümöle enthalten und/oder Konservierungsmittel enthalten sind, und es einen Gehalt an Eisen, Zink und Kupfer als Breitbandresonator aufweist, wobei dessen Anteil nicht größer ist als 3 Gewichtsprozent, bevorzugt unter 1 bis 0.01 Gewichtsprozent liegt, wobei das Präparat insbesondere als hydrophiles Öl, Öl-Alkohollösung, Emulsion, Salbe, Creme, Lotion, bevorzugt im sauren Bereich, ausgeführt ist.
- Sonnenschutz oder Bräunungspräparat, dadurch gekennzeichnet, dass es einen Gehalt an Eisen und Kupfer als Bräunungsfaktor aufweist, wobei dessen Anteil nicht größer ist als 1 Gewichtsprozent, bevorzugt unter 1 bis 0.01 Gewichtsprozent liegt, wobei das Präparat insbesondere als Emulsion, Salbe, Creme, Lotion, vorzugsweise im sauren Bereich formuliert ist und vorzugsweise einer Lösung natürlicher Salze und/oder eine saure Fraktion derselben enthält.(Das besondere hierbei ist, dass man durch das Verhältnis von Eisen und Kupfer einen unterschiedlichen der Braunton der Haut erreicht.)
- Sonnenschutz oder Bräunungspräparat, dadurch gekennzeichnet, dasses als Pflege-und/oder Wirkstoffe mindestens eine Substanz aus der Gruppe enthält, die Allantoin, Ascorbinsäure, Vitamin A, Sonnenschutzfilter, Zink, Aloe vera, Vitamin E, Panthothensäure, pflanzliche Öle, Jojobaöl, Olivenöl, essentielle Fettsäuren umfasst.
- Präparat, dadurch gekennzeichnet, dass der Breitbandresonator eine oder mehrere Komponenten aus der Gruppe enthält, die essentielle Mineralien, Metalle und Spurenelemente sowie nichtessentielle Metalle/Metallsalze und/oder Nichtmetalle, insbesondere Eisen, Zink, Kupfer, Mangan, Chrom, Molybdän, Vanadium, Nickel, Zinn, Silber, Gold, Jod, Arsen, Silizium, Calcium, Magnesium, Kalium, Natrium, Germanium. insbesondere Quarzsand, Gesteinsmehl, Mineralmehl, natürliche Salze, vorzugsweise Meersalz, Steinsalz oder Himalajasalz, Muschelkalk, Heilerde, Siliziumverbindungen, Kieselsäure, sowie Teile oder Auszüge der genannten natürlichen Stoffe und Zusammensetzungen umfasst.
- Präparat, dadurch gekennzeichnet, dass der Multiresonator Eisen und/oder eine Eisenverbindung umfasst.
- Präparat, dadurch gekennzeichnet, dass dieses mindestens ein, bevorzugt mehrere ferromagnetische Elemente und/oder eine Verbindung ferromagnetischer Elemente aus der Gruppe enthält, die Eisen, Kobalt und Nickel umfasst.
- Präparat, dadurch gekennzeichnet, dass es mindestens 5 verschiedene mineralische Elemente und/ oder Schwermetalle und/oder Verbindungen dieser Elemente enthält.
- Präparat, dadurch gekennzeichnet, dass es mindestens 10 verschiedene mineralische Elemente und/ oder Schwermetalle und/oder Verbindungen dieser Elemente enthält.
- Präparat, dadurch gekennzeichnet, dass es mindestens 15, bevorzugt 20 verschiedene mineralische Elemente und/oder Schwermetalle und/oder Verbindungen dieser Elemente enthält.
- Präparat, dadurch gekennzeichnet, dass dieses einen oder mehrere organische oder anorganische Gelbildner aus der Gruppe enthält, die Methylcellulosen, Alginate, Agar-Agar, Xanthan Gum, Johannisbrotkernmehl, Guakernmehl, Kieselsäure-Gel umfasst.
- Präparat, dadurch gekennzeichnet, dass die polare Trägersubstanz mindestens eine physiologischer Weise im Körper vorkommende Säuren, aus der Gruppe umfasst, die Salzsäure, Schwefelsäure, Phosphorsäure, Ascorbinsäure, Zitronensäure, Orotsäure, vorzugsweise 3- 5 oder mehr dieser Säuren umfasst.
- Präparat, dadurch gekennzeichnet, dass die Minuspolare Komponente als Kationen mindestens ein Element, vorzugsweise alle Elemente aus der Gruppe Kalium, Calcium, Natrium, Magnesium, insbesondere als Carbonate, Hydrogencarbonate, Hydroxide, Hydroxidcarbonate, Phosphate oder Chloride enthält.
- Präparat, dadurch gekennzeichnet, dass die Konzentration des oder der Breitbandresonatoren im Bereich von unter 10%, bevorzugt unter 5% insbesondere unter 1% liegt von.
- Präparat, dadurch gekennzeichnet, dass die Konzentration des oder der Breitbandresonatoren im Verdünnungs-Bereich homöopathischer Potenzen liegt, insbesondere zwischen D3 und D6.
- Präparat, dadurch gekennzeichnet, dass die Konzentration des oder der Breitbandresonatoren im Verdünnungs-Bereich homöopathischer Potenzen liegt, insbesondere zwischen D6 und D12 liegt.
- Präparat, dadurch gekennzeichnet, dass die Konzentration des oder der Breitbandesonatorenresonatoren im Verdünnungs-Bereich homöopathischer Mittel-und Hochpotenzen liegt, insbesondere zwischen D12 und D23 liegt.
- Präparat, dadurch gekennzeichnet, dass das Konzentrationsverhältnis von Wirkstoff zu Breitbandresonator größer ist als 5:1 ist oder größer als 10:1. (Oder umgekehrt)
- Präparat, dadurch gekennzeichnet, dass das Konzentrationsverhältnis von Wirkstoff zu Breitbandresonator größer als10:1 oder 100:1 oder 1000:1 ist. (Oder umgekehrt)
- Präparat, dadurch gekennzeichnet, dass das Konzentrationsverhältnis von Wirkstoff zu Breitbandresonator größer ist als 10000: 1 oder 100000:1 1 oder 1000000:1. (Oder umgekehrt)
- Präparat, dadurch gekennzeichnet, dass die polare Trägersubstanz und/oder die polare Multiresonator-Lösung einen pH-Wert im Bereich von unter 5,5 oder über 8,5 aufweist.
- Präparat oder Mehrkomponentenpräparat, dadurch gekennzeichnet, dass mindestens eine der polaren Trägersubstanzen oder bei verdünnten Systemen, insbesondere homöopathischen Zubereitungen, die Ausgangslösung mindestens einer der polaren Trägersubstanzen einen pH-Wert unter 2,5 bevorzugt 2 bis 0,5 und/oder über 11,0 bevorzugt 12,0-13,5 aufweist.
- Präparat, dadurch gekennzeichnet, dass die polare Trägersubstanz oder die Breitbandresonator-Lösung oder eine Mischung aus diesen einen Alkoholgehalt von 5 bis 70% aufweist.
- Präparat oder Mehrkomponentenpräparat, dadurch gekennzeichnet, dass mindestens eine der polaren Trägersubstanzen oder bei verdünnten Systemen, insbesondere homöopathischen Zubereitungen, die Ausgangslösung (sog. Urtinktur) mindestens einer der polaren Trägersubstanzen einen pH-Wert unter 3 und/oder über 10 bevorzugt aufweist.
- Präparat, dadurch gekennzeichnet, dass ein hohes Verhältnis von Polarität der Trägersubstanz(en) zur Konzentration des oder der Breitbandresonatoren eingestellt ist.
- Präparat, dadurch gekennzeichnet, dass eine oder mehrere arzneiliche und/oder kosmetische Wirkstoffe und/oder Stoffe zur Nahrungsergänzung enthalten sind.
- Präparat, dadurch gekennzeichnet, dass die Wirkstoffkomponente mineralischen, pflanzlichen oder tierischen Ursprungs ist oder synthetisch hergestellt ist.
- Präparat, dadurch gekennzeichnet, dass der der Anteil des Multiresonators kleiner als 10 Gewichstprozent, insbesondere kleiner als 1 Gewichtsprozent, vorzugsweise kleiner als 0,1 Gewichtsprozent ist.
- Präparat, dadurch gekennzeichnet, dass es als Flüssig- oder Halbflüssigpräparat, insbesondere als Lösung, Injektionslösung, Suspension, Gel oder Emulsion (W/O, O/W oder Mischemulsion) Emulgel, Creme, Salbe, Liniment, Hydrophiles Öl, Suppositorium (Zäpfchen) Verreibung (Trituration), Weich/Hartgelantinekapseln, Tabletten, Dragees, Globuli formuliert ,oder in Liposomen eingearbeitet ist.
- Präparat, dadurch gekennzeichnet, dass der Multiresonator und/oder der Wirkstoff und/oder das gesamte Präparat mittels eines homöopathischen Herstellungsverfahrens, insbesondere als Niedrig-, Mittel- oder Hochpotenz, vorzugsweise als Dezimalpotenz oder Centesimalpotenz in einer der bekannten Darreichungsformen gemäß DAB/Homöopathisches Arzneibuch hergestellt ist.
- Präparat, dadurch gekennzeichnet, dass es mindestens ein Schüsslersalz enthält.
- Präparat, dadurch gekennzeichnet, dass es mindestens eine Bachblüte enthält.
- Präparat zur Behandlung von Lebererkrankungen und der Unterstützung der Leberfunktion, dadurch gekennzeichnet, dass als Wirkstoff mindestens einen der pflanzlichen Arzneimittel aus der Gruppe Cheledonium, Lycopodium, Carduus marianus, Mandragora officinarum, Leptandra, in einer Konzentration von der Urtinktur bis D12 , vorzugsweise in Verbindung mit einer Resonatorenkonzentration im Bereich von der D1 bis D15 vorgesehen ist.
- Basisches Mineralstoffpräparat, dadurch gekennzeichnet, dass der pH-Wert zwischen 8 und12,5 liegt, bevorzugt zwischen 9 und 10,5 und der Mineralgehalt bis zu 60, bevorzugt zwischen 5 und 40 Gewichtsprozent liegt, und eine elektrolytisch gewonnene basische Fraktion einer natürlich vorkommenden und/oder nicht natürlich vorkommenden Mineralsalzlösung als Multiresonator und/oder Quarzsand enthält.
- Präparat, dadurch gekennzeichnet, dass der pH-Wert unter 5,5 liegt, bevorzugt zwischen 2 und 3,5 und der Gesamtmineralgehalt bevorzugt zwischen 0,5 bis 20 Gewichtsprozent liegt und dabei eine auf elektrolytischem Wege gewonnene saure Fraktion einer natürlich vorkommenden und/oder nicht natürlich vorkommenden Mineralsalzlösung als Multiresonator enthält.
- Präparat zur Verwendung als Badezusatz, dadurch gekennzeichnet, dass im saueren, neutralen oder basischen Bereich formuliert ist und einen Gehalt an Quarzsand aufweist.
- Präparat zur Verwendung als Badezusatz, dadurch gekennzeichnet, dass es aus einer sauren, puffernden Lösung besteht, insbesondere im pH-Bereich von 1,5 - 6 und dabei bevorzugt einen Gehalt an Fruchtsäueren und/oder Fruchtsalzen und/oder Carbonat/Bicarbonat und/oder Phosphat sowie mindestens 1 Metall aus der Gruppe enthält, die Eisen, Kupfer, Chrom, Mangan, Zink umfasst, wobei dieser vorzugsweise 1- 0,5 Gewichtsprozent nicht überschreitet.
- Mehrkomponenten-Präparat, dadurch gekennzeichnet, dass es mit mindestens eine Minus und eine Plus-Komponente enthält und vorzugsweise an Akupunturpunkten und Meridianen, sowie an Chakren eingesetzt wird und als Flüssig und/oder Halbflüssigpräparat, bevorzugt als Creme, Lotion oder Salbe formuliert ist.
- Mehrkomponenten-Präparat, dadurch gekennzeichnet, dass es mindestens eine Minus und Plus-Komponente enthält.
- Mehrkomponenten-Präparat, dadurch gekennzeichnet, dass dieses zur Anwendung als Akupunturpunkt und Meridiansalbe, sowie als Chakrensalbe verwendet wird.
- Mehrkomponenten-Salben-Präparat, dadurch gekennzeichnet, dass es mineralische und pflanzliche Wirkstoffe enthält und bevorzugt als polares, beispielsweise 2 mal 3 bis 2 mal 7 Salbenpräparate umfassendes Mehrkomponentenpräparat angeboten wird.
- Präparat zur Wundheilung, dadurch gekennzeichnet, dass dieses beispielsweise als Inhaltsstoffe Himalaiasalz/Steinsalz als Resonator (D8) Spurenelement-Lösung - sauer (D3/D4), Vitamine: A/B/C, Panthothensäure, Emulsion als O/W und W/O FormulierungG sowie Grapefruitkernextrakt enthält.
- Präparat, dadurch gekennzeichnet, dass es mindestens eine Nosode enthält.
- Präparat, dadurch gekennzeichnet, dass es eine oder mehrere Nosoden enthält, und diese mittels einem homöopathischen Verfahren hergestellt wurden.
- Präparat und/oder Präparatesystem, dadurch gekennzeichnet, dass dieses aus mindesten einem Wirkstoff und mehreren plus und/oder Minus Resonatoren besteht, welche individuell auf die Situation des Patienten bzw. den Verlauf der Krankheit angepasst werden können.
- Präparat zur Behandlung von Rheuma, dadurch gekennzeichnet, dass dieses als Wirkstoff einen oder mehrere Stoffe aus der Gruppe Bryonia, Rhus toxicodendron, Thuja, Carduus marianus, Cheledonium, Nux vomica, Spiraea ulmaria, Dulcamara, Clematis, Veratrum album, Sulfur, Staphisagria enthält, wobei diese im fertigen Präparat in der Konzentration von der Urtinktur bis D12 enthalten sind, vorzugsweise in Verbindung mit einer Resonatorenkonzentration im Bereich von D1 bis D12.
- Präparat, dadurch gekennzeichnet, dass es mindestens ein Organpräparat und/oder einen Organextrakt gewonnen aus Tierorganen enthält, bevorzugt aus der Gruppe die Hypophyse, Cerebrum, Schilddrüse, Thymusdrüse, Nebennieren, Gonaden, Lunge, Leber, Nieren, Dünndarm, Dickdarm, Herz, Haut umfasst.
- Präparat, dadurch gekennzeichnet, dass es mindestens ein Hormon und/oder Gewebshormon und/oder einen Neurotransmitter enthält, bevorzugt aus der Gruppe, die Thyroxin, Trijodthyronin, Gluco- und Mineralcorticoide, Serotonin, Dopamin, Heparin, Histamin, sowie männliche und weibliche Sexualhormone sowie Antikonzeptiva umfasst.
- Präparat, dadurch gekennzeichnet, dass es Vitamine und/oder Enzyme und/oder sekundäre Pflanzenstoffe enthält
- Kombinationspräparat, dadurch gekennzeichnet, dass es einen Multiresonator, einen Organischen Resonator und/oder ein Organpräparat und als Wirkstoff mindestens einen pflanzlichen Wirkstoff und/oder eine pflanzliche Tinktur enthält.
- Basisches Mehrkomponentenpräparat zur Durchführung einer mehrstufigen Supplementierung Therapie oder Diagnostik mit mehreren Präparaten und/oder Entsäuerung, insbesondere zur Therapie azidotischen Gewebes und/oder Ergänzung von Mineralstoffen , bei denen jedes eine unterschiedliche Konzentration an dem oder den Resonatoren und/oder ein unterschiedliches Polaritäts/Resonatorenkonzentrations-Verhältnis und/oder eine unterschiedliche Polarität aufweist. Der basische Resonator kann insbesondere Bestandteil eines Phytotherapeutikums sein, bevorzugt in Verbindung mit diuretisch wirksamen Substanzen (Nierenmittel, Bindegewebsmittel).
- Basisches Mehrkomponentenpräparat, dadurch gekennzeichnet, dass es als Gel ausgeführt ist und/oder einen Gehalt an Zink ausweist.bei denen jedes eine unterschiedliche Konzentration an dem oder den Resonatoren und/oder ein unterschiedliches Polaritäts/Resonatorenkonzentrations-Verhältnis und/oder eine unterschiedliche Polarität aufweist.
- Mehrkomponentenpräparat, dadurch gekennzeichnet, dass ein erstes Präparat zur Durchführung der ersten Stufe einer Therapie vorgesehen ist, welches eine niedrige Konzentration an dem oder den Resonatoren und/oder ein hohes Polaritäts/Resonatorenkonzentrations-Verhältnis aufweist und mindestens ein weiteres, vorzugsweise mehrere weitere Präparate, zur Durchführung weiterer Stufen einer Therapie vorgesehen sind, welche jeweils höhere Konzentrationen an dem oder den Resonatoren und/oder niedrigere Polaritäts/Resonatorenkonzentrations-Verhältnisse aufweisen.
- Mehrkomponentenpräparat, dadurch gekennzeichnet, dass ein erstes Präparat zur Durchführung der ersten Stufe einer Therapie vorgesehen ist, welches eine niedrige Konzentration an dem oder den Resonatoren und/oder ein hohes Polaritäts/Resonatorenkonzentrations-Verhältnis aufweist und mindestens ein weiteres, vorzugsweise mehrere weitere Präparate, zur Durchführung weiterer Stufen einer Therapie vorgesehen sind, welche jeweils höhere Konzentrationen an dem oder den Resonatoren und/oder niedrigere Polaritäts/Resonatorenkonzentrations-Verhältnisse der Stammlösung und/oder des Endprodukts sowie bevorzugt eine regelhafte Abstufung der polaren Trägersubstanz und/oder des Verdünnungsgrades aufweisen.
- Präparat, bei dem es sich um ein, bipolares Präparate-System für Diagnostik und Therapie handelt, bestehend aus je einer Reihe Plus-und Minus-Resonatoren, bei welchen im pH-Wert regelhaft abgestufte Lösungen als Stammlösung (STL) im Sinne einer homöopathischen "Urtinktur" zur Herstellung für bestimmte Potenzbereiche dienen. Minus Resonatoren
   STL-MR für D2-D3 pH 11,0
   STL-MR für D4-D8 pH 11,5
   STL-MR für D9-D12 pH 12,0
   STL-MR für D13-D15 pH 12,5
   STL-MR für D15-D18 pH 13,0
   Plus-Resonatoren
   STL-MR für D2-D3 pH 1,75
   STL-MR für D4-D8 pH 1,5
   STL-MR für D9-D12 pH 1,25,
   STL-MR für D13-D15 pH 1,0
   STL-MR für D15-D18 pH 0,75
- Resonatoren- Testsätze- Präparat zur energetischen Testung mittels geeigneter Verfahren (Elekto-Akupunktur, Radiästhesie/Kinesieologie), gekennzeichnet dadurch, dass dieser Testsatz aus Gefäßen, bevorzugt besteht Glas- oder Kunstoffröhrchen Röhrchen, in denen die jeweiligen Verdünnungsstufen und/oder Potenzen der Resonatoren und/oder Wirkstofflösungen und/oder Fertigpräparate enthalten sind.
- Mehrkomponentenpräparat, dadurch gekennzeichnet, dass ein erstes Präparat zur Durchführung der ersten Stufe einer Therapie vorgesehen ist, welches eine hohe Konzentration an dem oder den Resonatoren und/oder ein niedriges Polaritäts/Resonatorenkonzentrations-Verhältnis aufweist und mindestens ein weiteres, vorzugsweise mehrere weitere Präparate zur Durchführung weiterer Stufen einer Therapie vorgesehen sind, welche jeweils niedrigere Konzentrationen an dem oder den Resonatoren und/oder höhere Polaritäts/Resonatorenkonzentrations-Verhältnisse aufweisen.
- Mehrkomponentenpräparat zur Durchführung einer zweigleisigen Therapie mit zwei Präparaten, dadurch gekennzeichnet, dass ein Präparat eine saure polare Trägersubstanz und das andere Präparat eine basische polare Trägersubstanz aufweist.
- Mehrkomponentenpräparat zur Durchführung einer Wundheilungstherapie mit mindestens zwei Präparaten.
- Verfahren zur Herstellung eines Präparats, dadurch gekennzeichnet, dass ein homöopathisches Potenzierungsverfahren, insbesondere zur Erzeugung einer Niedrig-, Mittel- oder Hochpotenz, vorzugsweise Dezimalpotenz oder Centesimalpotenz und/oder ein Energetisierungsverfahren, insbesondere gemäß dem Bioresonanz-und/oder Magnetisierungsverfahren durchgeführt wird.
- Verfahren zur Herstellung eines Präparats, dadurch gekennzeichnet, dass der Wirkstoff und/oder der Breitbandresonator und die polare Trägersubstanz getrennt voneinander verdünnt und/oder einem homöopathischen Potenzierungsverfahren unterzogen werden und danach wieder zu einem Präparat zusammengeführt werden, wobei bevorzugt eine homöopathischen Aufbereitung (Verschüttelung) durchgeführt wird.
- Verfahren zur Herstellung eines Präparats, dadurch gekennzeichnet, dass die Ausgangslösungen (homöopathischen Urtinkturen) von Breitbandresonator und polarer Trägersubstanz die gleiche Polarität ggf. in unterschiedlicher Ausprägung aufweisen.
- Verfahren zur Herstellung eines Präparats, dadurch gekennzeichnet, dass die Ausgangslösungen (homöopathischen Urtinkturen) von Breitbandresonator und polarer Trägersubstanz einen unterschiedlichen pH-Wert und/oder eine unterschiedliche Polarität aufweisen.
- Verfahren zur Herstellung eines Präparats, dadurch gekennzeichnet, dass der Wirkstoff und/oder der Breitbandresonator und die polaren Trägersubstanz getrennt voneinander in unterschiedlichen Maße verdünnt und/oder potenziert werden, wobei der Verdünnungsgrad jeweils auf einem anderen Algorithmus beruht.
- Präparat, dadurch gekennzeichnet, dass der Wirkstoff und/oder der Breitbandresonator und die polaren Trägersubstanz getrennt voneinander in unterschiedlichen Maße verdünnt und/oder potenziert werden, wobei der Verdünnungsgrad jeweils auf einem anderen Algorithmus beruht.
- Mehrkomponenten-Präpart, dadurch gekennzeichnet, dass der Wirkstoff und/oder der Breitbandresonator und die polare Trägersubstanz getrennt voneinander in unterschiedlichen Maße verdünnt und/oder potenziert werden, wobei der Verdünnungsgrad jeweils auf einem anderen Algorithmus beruht.
- Verfahren zur Herstellung eines Präparats, dadurch gekennzeichnet, dass der Breitbandresonator und die polaren Trägersubstanz getrennt voneinander in unterschiedlichen Schritten verdünnt werden, wobei diese jeweils auf einem anderen Algorhythmus beruhen und dabei so gewählt werden, dass diese mit jedem Verdünnungs- und/oder Potenzierungsschritt zu einer relativen stärkeren Verdünnung des Breitbandresonators führen.
- Verfahren zur Herstellung eines Mehrkomponentenpräparats, dadurch gekennzeichnet, dass der Breitbandresonator und die polaren Trägersubstanz getrennt voneinander in unterschiedlichen Schritten verdünnt werden, wobei die Differenz der Konzentration der enthaltenen Stoffe mit zunehmender Verdünnung zu oder abnimmt.
- Mehrkomponenten-Präpart nach, dadurch gekennzeichnet, dass der Breitbandresonator und die polaren Trägersubstanz getrennt voneinander in unterschiedlichen Schritten verdünnt werden, wobei die Differenz der Konzentration der enthaltenen Stoffe mit zunehmender Verdünnung der einzelnem Komponenten bevorzugt in nichtlinearer Weise zunimmt und hieraus eine relative Zunahme der Polarität resultiert.
- Verfahren zur Herstellung eines Präparats, dadurch gekennzeichnet, dass der Breitbandresonator und die polaren Trägersubstanz getrennt voneinander in unterschiedlichen Schritten verdünnt werden, wobei die Differenz der Konzentration der enthaltenen Stoffe bevorzugt in nichtlinearer Weise abnimmt und hieraus eine relative Abnahme der Polarität resultiert.
- Präparat, dadurch gekennzeichnet, dass dieses als isotone Lösung für Injektionen vorliegt.
- Verfahren zur Herstellung eines Präparats, dadurch gekennzeichnet, dass der Breitbandresonator getrennt von der polaren Trägersubstanz in unterschiedlichen Schritten verdünnt oder potenziert wird.
- Präparat, dadurch gekennzeichnet, dass es mittels eines Informierungs- und /oder Energetisierungsverfahrens und/oder Aufschwingungsverfahren, insbesondere gemäß einem Bioresonanzverfahren und/oder radionischem Verfahren unter Verwendung eines Bioresonanzgerätes beispielsweise mittels eines Rayonex-, Vega-, Mora-Gerätes, und/oder einem Magnetisierungsverfahren hergestellt ist.
- Präparat, dadurch gekennzeichnet, dass es im Rahmen eines eines Informierungs- und /oder Energetisierungsverfahrens und/oder Aufschwingungsverfahren und/oder gemäß einem Bioresonanzverfahren und/oder radionischem Verfahren unter Verwendung eines Bioresonanzgerätes beispielsweise mittels eines Rayonex-, Vega-, Mora-Gerätes, und/oder einem Magnetisierungsverfahren eingesetzt wird und/ oder in eines dieser Geräte eingebaut ist.
- Verfahren, dadurch gekennzeichnet, dass die Wechselwirkung zwischen spezifischen Wirkstoff(en) und dem oder den Resonatoren und/oder die energetischen Verhältnisse der Gewebe und Strukturen berücksichtigt werden.
- Präparat, dadurch gekennzeichnet, dass der Multiresonator und/oder die Wirkstoffe zur Vermeidung von Wechselwirkungen in Liposomen eingebracht sind.
- Verfahren, dadurch gekennzeichnet, dass die Resonatorkonzentration der Wirkstoffkonzentration angepasst wird, insbesondere im Bereich von wenigen Dezimalpotenzen über oder unter der Wirkstoffpotenz.
- (Mehr-)Komponenten-Präparat bzw. Verfahren, dadurch gekennzeichnet, dass die Resonatorkonzentration bei nicht homöopathischen Wirkstoffen umso höher gewählt wird, je toxischer der Wirkstoff ist und umso niedriger bei homöopathischen Wirkstoffen.
- Verfahren, dadurch gekennzeichnet, dass die Polarität umso höher eingestellt wird, je höher die Konzentration des Resonators ist.
- Verfahren, dadurch gekennzeichnet, dass die Polarität umso niedriger eingestellt wird, je höher die Konzentration des Resonators ist.
- Verfahren, dadurch gekennzeichnet, dass ein elektrolytisches Verfahren zur Herstellung einer Pluspolaren und/oder Minuspolaren Fraktion von Salzlösungen, insbesondere auch Lösungen natürlicher Salze wie beispielsweise Meersalz und/oder Steinsalz, Himalaia-Salz verwendet wird.
- Vorrichtung/Nadel zur Durchführung der Akkupunktur, dadurch gekennzeichnet, dass diese teilweise mit einem Breitbandresonator beschichtet ist.
- Vorrichtung/Nadel zur Durchführung der Akupunktur, wobei diese einen oder mehrere polare Minus oder plus Resonatoren und/oder spezifische planzliche oder tierische Präparate enthalten. (Beispielhafte Ausführungsformen siehe Fig. 1 und 2)
- Herstellungsverfahren für neutralen Resosator, dadurch gekennzeichnet, dass dieses erst auf einen basischen pH-Wert und dann mittels Zugabe von mindestens einer Säure auf einen neutralen pH-Wert (ca. 7) eingestellt wird.
- Verfahren, dadurch gekennzeichnet, dass in der Minuskomponente oder in der basischen Komponente auf die Verwendung von Elementen oder Verbindungen aus der Gruppe die Eisen, Kupfer, Chrom, oder Mangan umfasst verzichtet wird, weil diese die energetisch-physikalischen Eigenschaften des Präparats stören können.
- Set aus mindestens 2 Substanzen der Gruppe die einen oder mehrere Zucker, Säuren, Mineralsalze, Kochsalz, Pflanzenzubereitungen, Heilpflanzenzubereitungen sowie insbesondere deren wässrige Lösungen umfasst, wobei das Set bevorzugt Substanzen und/oder Substanzmischungen in Glasröhrchen/Ampullen etc. in unterschiedlichen Konzentrationen enthält und dieses Set insbesondere im Rahmen der Bioresonanztherapie zu diagnostischen Zwecken und individuellen, hinsichtlich der Resonanz optimierten Zusammenstellungen dient.
- Die Heilpflanzen umfassen insbesondere Aloe, Birkenblätter, Christrose (Schwarze Nießwurz),Enzian, Hauhechel, Hirtentäschel Ingwer/Wilder Ingwer, Kalmus, Kamille, Kurkuma, Liebstöckel, Löwenzahn, Lungenkraut, Mariendistel, Mistel, Myrrhe, Nelken, Nelkenwurz, Odermenning, Passionsblume, Petersilienwurzel, Pfefferminz, Pfeffer/Capsicum, Raute, Rettichsamen, Ringelblume, Rosmarin,Salbei, Schachtelhalm, Schafgarbe,Schlüsselblume, Seifenkraut, Senfsamen, Spirea, Ulmaria, Spirulina, Spitzwegerich, Sternanis, Süßholz, Taraxacum, Tausendgüldenkraut, Thymian, Veilchen, Wacholder, Walnuss, Weihrauch, Weißdorn, Wermut, Ysop, Zimtrinde.
- Die Organpräparate können insbesondere für folgende Organe, Körperteile oder Therapien vorgesehen sein oder hierfür bekannte Wirkstoffe umfassen:
   1. Zirbeldrüse
   2. Großhirn
   3. Gehirn Pons
   4. Hypothalamus
   5. Hypophyse
   6. Kleinhirn
   7. Auge 1 *(oculus totalis)*
   *8. Auge 2 (corneal corpus vitreum*/*lens*/ *musculus oculi)*
   *9.* Auge 3 *(nervus opticus*/*Retina*)
   10. Mittelohr (Eustachische Röhre)
   *11.* Nase *(nervus olfaktorius*/*mucosa nasalis)*
   12. Tonsillen
   13. Speicheldrüse
   14. Schilddrüse
   15. Nebenschilddrüse
   16. Kehlkopf
   17. Thymusdüse
   18. Herz
   19. Bronchien
   20. Lunge
   21. Zahn *(dens* /*Pulpa dentis*/*Gingiva)*
   22. Mamma
   23. Magen
   24. Dünndarm *(Duodenum*/*Jejunum)*
   25. Bauchspeicheeldrüse
   26. Milz
   27. Leber
   28. Gallenblase
   29. Dickdarm *(Coecum und Colon)*
   30. Nebenniere
   31. Niere *(Niere*/*Pyelon)*
   32. Ovarien
   33. Uterus
   34. Placenta
   35. Prostata
   36. Testis
   *37.* Harnblase *(Harnblase*/*Harnröhre)*
   38. Symphaticus
   *39.* Haut *(Epidermis*/*Cutis)*
   40. Arterien
   41. Venen
   42. Blut
   43. Lymphknoten
   44. Rückenmark *(medulla spinalis*/*medulla oblongata)*
   45. Knochen
   46. Knorpel
   *47.* Wirbelsäule *(Disci intervertebrales)*
   48. Zellerneuerung

### KURZE BESCHREIBUNG DER ZEICHNUNG

Die beigefügten Zeichnungen zeigen in rein schematischer Weise in
Fig. 1 eine Darstellung einer ersten Anordnung von Resonatoren an Akkupunktur-Nadeln; und in
Fig. 2 eine Darstellung einer zweiten Anordnung von Resonatoren an Akkupunktur-Nadeln.

### BEVORZUGTE AUSFÜHRUNGSFORMEN

Die praktische Anwendung der vorliegenden Erfindung wird anhand nachfolgender Anwendungsbeispiele beschrieben:

Zur Formulierung von Präparaten werden zwei Formen von Multiresonatoren eingesetzt:
Minus (basischer)-Resonator mit einem Element/Verbindung aus der Gruppe Calcium, Magnesium, Kalium, Natrium, Barium,
Plus (saurer) - Resonator mit Metallen aus der Gruppe der Schwer- und Leichtmetalle.

### Beispiel 1:

Jodhaltiges Präparat, z.B. Schilddrüsen Präparat mit Thyroxin und/oder Trijodthyrodin. Hier dient der SSM Multiresonator insbesondere der Verringerung der Toxizität.

### Beispiel 2:

Präparat zur Behandlung von Leberstörungen und/oder zur Anregung des Leberstoffwechsels. In diesem Fall ist, wie in vielen anderen Fällen auch, eine Formulierung sowohl im sauren wie auch im basischen Bereich sinnvoll, in Abhängigkeit von der Störung und der damit verbundenen Stoffwechsellage. Denkbar ist auch, die phasenweise Anwendung mit unterschiedlichen pH-Werten im sauren und basischen Bereich. Spezifische erfindungsgemäße Leber-Präparate können beispielsweise wie folgt aufgebaut sein:
Basisches Leberpräparat mit basischem Multiresonator mit einer Magnesium-, Kalium-, Calcium-Verbindung mit einem oder mehreren in der Lebertherapie bekannten Phythotherapeutica aus der Gruppe Cheledonium, Lycopodium, Carduus marianus, Taraxacum officinale. Da Lebermittel ohnehin basisch sind, ist die erfindungsgemäße Formulierung dadurch gekennzeichnet, dass der pH-Wert noch erhöht wird durch den basischen Multiresonator (bei homöopathischen Zubereitungen betrifft dies die Ausgangslösung des Präparats, sog. Urtinktur und/oder den Multiresonator).
Saures/Plus Leberpräparat mit den gleichen spezifischen Pflanzenwirkstoffen und einem Breitbandresonator, bestehend aus Schwer- und/oder Leichtmetallen und/oder deren Salze/Kolloide. Anzuwenden sind diese dann je nach Stoffwechsellage oder energetischer Situation. Denkbar ist auch dass ein Patient mit beiden Varianten therapiert wird.

### Beispiel 3:

Nierenpräparat im sauren oder basischen Bereich. Das basische Mittel dient mehr zur Steigerung der Entgiftungsleistung, das saure Mittel zur Stärkung der Energetik der Niere zur Verbesserung der Rückresorptionsprozesse, welche sich auf indirekte Weise positiv auf die Entgiftungstätigkeit auswirken.

### Beispiel 4:

Präparat zur Behandlung von Traumen und/oder Wundzuständen. Hier ist es wichtig den gestörten Stoffwechsel rasch zu normalisieren, um Resorption und Wirkung der spezifischen Wirkstoffe zu erreichen, die einer Wirkungsentfaltung entgegenstehen. Bei manchen Stoffen die bekanntermaßen leicht Allergien oder Unverträglichkeiten auslösen, wie z. B. Arnica montana (Bergwohlverleih) erlebt man eine Reduktion der allergenen Wirkung. Da Trauma-und Wundheilpräparate insbesondere für den Akutbereich vorteilhaft bevorzugt im sauren Milieu darzustellen sind, ist die dämpfende Wirkung des Multiresonators von besonderer Bedeutung, da im sauren Bereich potentiell toxische Wirkungen/Nebenwirkungen der Wirkstoffe wegen der destabilisierenden Wirkung der Säuren auf die Membranen, sehr viel stärker hervortreten.

### Solche Formulierungen können beispielsweise sein:

Wundsalbe mit einem pH von 3-4 mit Panthothensäure, Dexpanthenol, Vitamin A, Vitamin C, einem Zinksalz (Zinkchlorid, Zinksulfat),
Traumasalbe mit pH-2,5-3,5 mit einem Gehalt an Arnica, Beinwell, Symphitum, Kamille, Campher.
Traumasalbe mit ph3-4 mit einem (nichtsteroidem) Antiphlogisticum (z.B. Diclophenac).

### Beispiel 5:

Cortisonhaltige Hautsalben und Hautcremes. Da Cortison erhebliche Nebenwirkungen in Form von schweren trophischen Hautschäden (Pergamenthaut) verursachen kann, ist eine Verringerung der Nebenwirkung von besonderer Bedeutung. Diese kann durch den Einsatz sowohl eines Plus- wie eines Minus-Resonators erfolgen, und sich nach der Indikation richten.

### Wundsalbe mit Enzymen

### Beispiel 6:

Parfümhaltige Zubereitungen sind auch ein Anwendungsbereich der erfindungsgemäßen Zubereitung, da Parfüms bekanntermaßen ein vergleichsweise hohes allergenes Potential besitzen, welches Tests zufolge sogar noch höher ist, als das von Konservierungsstoffen. Ziel ist hier vor allem durch den Einsatz der erfindungsgemäßen Breitbandesonatoren mit Metallsalzen und/oder Nichtmetallen (Eisen, Zink, Kupfer, Mangan, Molybdän, Selen, Silizium (Kieselerde) eine Abschwächung des toxischen bzw. allergenen Potentials zu erreichen.

Wesentlich ist hier die Erkenntnis, dass dieser überraschende Effekt bereits mit vergleichsweise sehr geringen Konzentrationen erreicht wird, so dass die Produkteigenschaften nicht nachteilig beeinflusst werden.Eine bevorzugte Ausführung des erfindungsgemäßen Präparats ist die Formulierung als Emulsion oder Emulgel, da eine Kombination von Multiresonator und Parfümölen eine Verbindung von Öl/Fett und Wasser erfordern kann. Hierfür eignen sich handelsübliche Emulgatoren und/oder Lösungsvermittler.

### Beispiel 7:

Anti-Pilz-Präparat als basisches Präparat mit pH> 8, zur Unterstützung einer gesunden Darmflora, reduziert die pathologische Fehlbesiedlung (z.B. candida albicas); die Hauptwirkstoffe sind pflanzliche Antimykotika wie Grapefruitkernextraxt oder Olivenblätterextrakt u.a. (Prinzipiell kann auch ein antimykotisches Monopräparat wie Nystatin durch entsprechende pH-Einstellung optimiert werden).

Ein weiteres Einsatzgebiet ist ein Immun-Präparat zur Behandlung akuter und chron. rezidivierender Infekte.

### Beispiel 8: Präparate-Set

**Tabelle 1**

| **Konzentration** | **Resonatoren und Co-Faktoren für die Resonanz** | | | | |
|---|---|---|---|---|---|
| 25% | Säure | Zucker | Mineralsalzlösung | Phytomischung | Säuremischung |
| 50% | Säure | Zucker | Mineralsalzlösung | Phytomischung | Säuremischung |
| 75% | Säure | Zucker | Mineralsalzlösung | Phytomischung | Säuremischung |
| 100% | Säure | Zucker | Mineralsalzlösung | Phytomischung | Säuremischung |

Tabelle 1 zeigt eine beispielhafte Ausführung eines erfindungsgemäßen diagnostischen und therapeutischen Präparate-Sets dessen Besonderheit ist, dass dieses eine Matrix abbildet wie sie in besonderer Weise zur Optimierung und Individualisierung von Phytho-Rezepturen geeignet ist, insbesondere auch für Zubereitungen der traditionellen chinesischen Medizin (TCM). Ein Vorteil der speziellen Ausgestaltung des erfindungsgemäßen Sets ist hier die Möglichkeit einer systematischen quantitativen und qualitativen Formulierung und individuellen Anpassung von Präparaten unter Einbeziehung der im System der TCM bedeutsamen Geschmackskriterien.

Das Präparate-Set umfasst somit 20 Fläschchen oder dergleichen mit 5 unterschiedlichen Substanzen in 4 verschiedenen Konzentrationen, die der Therapeut je nach Ergebnis der Bioresonanzmessungen entsprechend einsetzen kann.

Obwohl die Erfindung anhand spezifischer Beispiele dargestellt worden ist, ist für den Fachmann klar und verständlich, dass die Erfindung im Rahmen des Schutzbereichs der beigefügten Ansprüche geändert und abgewandelt werden kann. Insbesondere sind Kombinationen einzelner Merkmale oder das Weglassen bestimmter Merkmale möglich, ohne den Schutzbereich zu verlassen. Der Offenbarungsgehalt umfasst insbesondere alle möglichen Kombinationen von beschriebenen Substanzen im Rahmen der Erfindung, ohne dass es erforderlich wäre jede Kombination einzeln zu beschreiben.

## Patentansprüche

1. Präparat zur Unterstützung der Aufnahme und/oder Steigerung der Wirkung von Wirkstoffen, insbesondere arzneilichen Wirkstoffen, oder Nahrungsergänzungsmitteln in einem menschlichen oder tierischen Körper, insbesondere unter Vermeidung von Nebenwirkungen,
**dadurch gekennzeichnet, dass**
mindestens eine polare Trägersubstanz und mindestens ein Breitbandresonator für den menschlichen oder tierischen Körper enthalten sind.

2. Präparat zur Unterstützung der Aufnahme und/oder Steigerung der Wirkung von Wirkstoffen, insbesondere arzneilichen Wirkstoffen, oder Nahrungsergänzungsmitteln in einem menschlichen oder tierischen Körper, insbesondere unter Vermeidung von Nebenwirkungen,
**dadurch gekennzeichnet, dass**
mindestens eine polare, insbesondere monopolare, vorzugsweise zumindest ursprünglich saure oder basische Trägersubstanz und mindestens ein Wirkstoff, vorzugsweise ein natürlicher oder synthetischer, insbesondere mineralischer, pflanzlicher oder tierischer Wirkstoff für den menschlichen oder tierischen Körper enthalten sind.

3. Präparat nach Anspruch 1 oder 2,
**dadurch gekennzeichnet, dass**
die polare Trägersubstanz eine saure, basische oder alkoholische Lösung oder eine Verdünnung einer derartigen Lösung aus einem oder mehreren polaren Lösungsmitteln, insbesondere auf Wasserbasis und/oder Alkoholbasis ist, wobei insbesondere die Verdünnung so stark sein kann, dass die saure, basische oder alkoholische Komponente nicht mehr nachweisbar ist.

4. Präparat zur Vermeidung toxischer Wirkungen und/oder Verbesserung der Vertäglichkeit von Substanzen, die auf den menschlichen oder tierischen Körper einwirken, durch Beimischung,
**dadurch gekennzeichnet, dass**
mindestens ein Breitbandresonator enthalten ist.

5. Präparat nach einem der vorhergehenden Ansprüche,
**dadurch gekennzeichnet, dass**
der Breitbandresonator eine oder mehrere Komponenten aus der Gruppe umfasst, die Mineralien, Spurenelemente, Emulgatoren, Gelbildner, essentielle oder nichtessentielle Metalle/Metallsalze und/oder Nichtmetalle, insbesondere Quarzsand, Gesteinsmehl, Mineralmehl, natürliche Salze, vorzugsweise Meersalz, Steinsalz oder Himalajasalz, Muschelkalk, Heilerde, Siliziumverbindungen, Methylcellulose, Alginate, Agar-Agar, Johannisbrotkernmehl, Guakernmehl, Kieselsäure, Eisen, Zink, Kupfer, Mangan, Chrom, Molybdän, Vanadium, Nickel, Zinn, Silber, Gold, Jod, Arsen, Silizium, Calcium, Magnesium, Kalium, Natrium, Germanium, sowie Auszüge und Verbindungen dieser Stoffe umfasst.

6. Präparat nach einem der vorhergehenden Ansprüche,
**dadurch gekennzeichnet, dass**
der Breitbandresonator Eisen und/oder mindestens eine Eisenverbindung umfasst.

7. Präparat nach einem der vorhergehenden Ansprüche,
**dadurch gekennzeichnet, dass**
es mindestens eine, bevorzugt mehrere ferromagnetische Komponenten und/oder Verbindungen, insbesondere umfassend ein Element aus der Gruppe, die Eisen, Kobalt oder Nickel enthält.

8. Präparat nach einem der vorhergehenden Ansprüche,
**dadurch gekennzeichnet, dass**
mindestens jeweils ein Plus- und Minus-Resonator enthalten ist.

9. Präparat nach einem der vorhergehenden Ansprüche,
**dadurch gekennzeichnet, dass**
es mindestems 5, vorzugsweise 10, insbesondere 15 verschiedene Komponenten und/oder Verbindungen dieser Komponenten, insbesondere mineralische Komponenten und/oder
Schwermetalle und/oder Verbindungen daraus enthält.

10. Präparat nach einem der vorhergehenden Ansprüche,
**dadurch gekennzeichnet, dass**
die polare Trägersubstanz mindestens eine physiologischerweise im Körper vorkommende Säure, insbesondere Salzsäure, Schwefelsäure, Phosphorsäure, Ascorbinsäure, Zitronensäure, Orotsäure, oder Base, insbesondere auf Basis von Natriumbicarbonat oder Kaliumcarbonat, umfasst.

11. Präparat nach einem der vorhergehenden Ansprüche,
**dadurch gekennzeichnet, dass**
die Minuspolare Komponente als Kationen mindestens ein Element, vorzugsweise alle Elemente aus der Gruppe Kalium, Calcium, Natrium, Magnesium, insbesondere als Carbonate, Hydrogencarbonate, Hydroxide, Hydroxidcarbonate oder Chloride enthält.

12. Präparat nach einem der vorhergehenden Ansprüche,
**dadurch gekennzeichnet, dass**
die Konzentration des oder der Breitbandresonatoren im Bereich von 0 bis 10 Vol.%, insbesondere von 0 bis 5 Vol.%, vorzugsweise 0 bis 1 Vol.% liegt oder kleiner 10 Gew.%, vorzugsweise kleiner 1 Gew.%, insbesondere kleiner 0,1 Gew.% ist.

13. Präparat nach einem der vorhergehenden Ansprüche,
**dadurch gekennzeichnet, dass**
die Konzentration des oder der Breitbandresonatoren im Verdünnungsbereich homöopathischer Potenzen liegt, insbesondere im Bereich von D3 bis D23, vorzugsweise D3 bis D6 oder D6 bis D 12 oder D12 bis D23.

14. Präparat nach einem der vorhergehenden Ansprüche,
**dadurch gekennzeichnet, dass**
die polare Trägersubstanz und/oder das Präparat und/oder die Ausgangssubstanz bei verdünnten Präparaten einen pH-Wert im Bereich unter 5,5, vorzugsweise unter 3,0, höchst vorzugsweise unter 2,5, insbesondere 0,5 bis 2 oder über 8,5, vorzugsweise über 10,0, höchst vorzugsweise über 11,0, insbesondere 12,0 bis 13,5 aufweist.

15. Präparat nach einem der vorhergehenden Ansprüche,
**dadurch gekennzeichnet, dass**
die polare Trägersubstanz und/oder das Präparat und/oder die Ausgangssubstanz bei verdünnten Präparaten eine alkoholische Komponente mit einer Konzentration von 5 bis 70 Vol.% aufweist.

16. Präparat nach einem der vorhergehenden Ansprüche,
**dadurch gekennzeichnet, dass**
ein hohes Verhältnis von Polarität der Trägersubstanz(en) zur Konzentration des oder der Breitbandresonatoren eingestellt ist.

17. Präparat nach einem der vorhergehenden Ansprüche,
**dadurch gekennzeichnet, dass**
eine oder mehrere arzneiliche und/oder kosmetische Wirkstoffe und/oder Nahrungsergänzungsmittel enthalten sind.

18. Präparat nach einem der vorhergehenden Ansprüche,
**dadurch gekennzeichnet, dass**
die Wirkstoffkomponente pflanzlichen oder tierischen Ursprungs ist oder synthetisch hergestellt ist.

19. Präparat nach einem der vorhergehenden Ansprüche,
**dadurch gekennzeichnet, dass**
das Konzentrationsverhältnis von Wirkstoff oder Wirkstoffkomponenten zu dem oder den Breitbandresonatoren größer 5:1, insbesondere größer 10:1, vorzugsweise größer 100:1, höchst vorzugsweise größer 1 000:1, bevorzugt 10 000:1 1 und höchst bevorzugt 100 000:1 oder 1000 000:1 ist.

20. Präparat nach einem der vorhergehenden Ansprüche,
**dadurch gekennzeichnet, dass**
der Gesamtmineralstoffgehalt kleiner als 5 Gewichtsprozent, insbesondere kleiner als 1 Gewichtsprozent, vorzugsweise kleiner als 0,1 Gewichtsprozent ist.

21. Präparat nach einem der vorhergehenden Ansprüche,
**dadurch gekennzeichnet, dass**
es als Flüssig- oder Halbflüssigpräparat, insbesondere als Lösung, Injektionslösung, Suspension, Gel, Emulsion, Emulgel, Creme, Salbe, Liniment, hydrophiles Öl, Zäpfchen, Verreibung, Weich- oder Hartgelantinekapseln, Tabletten, Dragees, Globuli oder in sonstiger Kapselform formuliert oder in Liposomen eingearbeitet ist.

22. Präparat nach einem der vorhergehenden Ansprüche,
**dadurch gekennzeichnet, dass**
dieses einen oder mehrere organische oder anorganische Gelbildner aus der Gruppe enthält, die Methylcellulosen, Alginate, Agar-Agar, Xanthan Gum, Johannisbrotkernmehl, Guakernmehl, Kieselsäure-Gel umfasst.

23. Präparat nach einem der vorhergehenden Ansprüche,
**dadurch gekennzeichnet, dass**
es und/oder eine seiner Komponenten mittels eines homöopathischen Herstellungsverfahrens, insbesondere Potenzierungsverfahrens, insbesondere als Niedrig-, Mittel- oder Hochpotenz, vorzugsweise als Dezimalpotenz oder Centesimalpotenz insbesondere in einer Darreichungsform nach DAB oder homööpathischem Arzneibuch hergestellt ist.

24. Präparat nach einem der vorhergehenden Ansprüche,
**dadurch gekennzeichnet, dass**
es mittels eines Informierungs- und/oder Energetisierungs- und/oder Aufschwingungsverfahrens, insbesondere gemäß einem Bioresonanzverfahren und/oder mittels eines radionischen Verfahrens unter Verwendung eines Bioresonanzgeräts und/oder mittels eines Magnetisierungsverfahrens hergestellt ist.

25. Präparat nach einem der vorhergehenden Ansprüche,
**dadurch gekennzeichnet, dass**
es ein oder mehrere ätherische Öle, insbesondere auch Parfümöle und/oder Konservierungsmittel enthält, und es einen Gehalt an Eisen, Zink und Kupfer als Breitbandresonator aufweist, wobei dessen Anteil nicht größer ist als 3 Gewichtsprozent, bevorzugt unter 1 bis 0.01 Gewichtsprozent liegt, wobei das Präparat insbesondere als hydrophiles Öl, Öl-Alkohollösung, Emulsion, Salbe, Creme, Lotion, bevorzugt im sauren Bereich, ausgeführt ist.

26. Präparat nach einem der vorhergehenden Ansprüche, insbesondere zur Verwendung als Sonnenschutz oder Bräunungsmittel,
**dadurch gekennzeichnet, dass**
es einen Gehalt an Eisen und Kupfer als Bräunungsfaktor aufweist, wobei dessen Anteil nicht größer ist als 1 Gew.%, bevorzugt unter 1 bis 0.01 Gew.% liegt, wobei das Präparat insbesondere als Emulsion, Salbe, Creme, Lotion, vorzugsweise im sauren Bereich formuliert ist und vorzugsweise einer Lösung natürlicher Salze und/oder eine saure Fraktion derselben enthält.

27. Präparat nach einem der vorhergehenden Ansprüche, insbesondere nach Anspruch 26
**dadurch gekennzeichnet, dass**
es als Pflege- und/oder Wirkstoffe mindestens eine Substanz aus der Gruppe enthält, die Allantoin, Ascorbinsäure, Vitamin A, Sonnenschutzfilter, Zink, Aloe vera, Vitamin E, Panthothensäure, pflanzliche Öle, Jojobaöl, Olivenöl, essentielle Fettsäuren umfasst.

28. Präparat nach einem der vorhergehenden Ansprüche,
**dadurch gekennzeichnet, dass**
es mindestens ein Schüsslersalz und/oder mindestens eine Bachblüte umfasst.

29. Präparat nach einem der vorhergehenden Ansprüche,
**dadurch gekennzeichnet, dass**
der pH-Wert zwischen 8 und12,5 liegt, bevorzugt zwischen 9 und 10,5 und der Mineralgehalt bis zu 60 Gew.%, bevorzugt zwischen 5 und 40 Gew.% liegt, und es eine elektrolytisch gewonnene basische Fraktion einer natürlich vorkommenden und/oder nicht natürlich vorkommenden Mineralsalzlösung als Breitbandresonator und/oder Quarzsand enthält.

30. Präparat nach einem der vorhergehenden Ansprüche,
**dadurch gekennzeichnet, dass**
der pH-Wert unter 5,5 liegt, bevorzugt zwischen 2 und 3,5 und der Gesamtmineralgehalt bevorzugt zwischen 0,5 bis 20 Gewichtsprozent liegt und es eine auf elektrolytischem Wege gewonnene saure Fraktion einer natürlich vorkommenden und/oder nicht natürlich vorkommenden Mineralsalzlösung als Breitbandresonator enthält.

31. Präparat nach einem der vorhergehenden Ansprüche,
**dadurch gekennzeichnet, dass**
es mindestens ein Organpräparat und/oder ein Organextrakt gewonnen aus Tierorganen umfasst, bevorzugt aus der Gruppe die Hypophyse, Cerebrum, Schilddrüse, Thymusdrüse, Nebennieren, Gonaden, Lunge, Leber, Nieren, Dünndarm, Dickdarm, Herz, Haut umfasst.

32. Präparat nach einem der vorhergehenden Ansprüche,
**dadurch gekennzeichnet, dass**
es mindestens ein Hormon und/oder ein Gewebshormon und/oder einen Neurotransmitter umfasst, bevorzugt aus der Gruppe, die Thyroxin, Trijodthyronin, Gluco- und Mineralcorticoide, Serotonin, Dopamin, Heparin, Histamin, sowie männliche und/oder weibliche Sexualhormone sowie Antikonzeptiva umfasst.

33. Präparat nach einem der vorgehenden Ansprüche,
**dadurch gekennzeichnet, dass**
es Vitamine und/oder Enzyme und/oder sekundäre Pflanzenstoffe enthält

34. Präparat nach einem der vorgehenden Ansprüche,
**dadurch gekennzeichnet, dass**
es einen Multiresonator, einen organischen Resonator und/oder ein Organpräparat und als Wirkstoff mindestens einen pflanzlichen Wirkstoff und/oder eine pflanzliche Tinktur umfasst.

35. Präparat nach einem der vorhergehenden Ansprüche, insbesondere zur Verwendung als Badezusatz,
**dadurch gekennzeichnet, dass**
es im saueren, neutralen oder basischen Bereich formuliert ist und einen Gehalt an Quarzsand aufweist.

36. Präparat nach einem der vorhergehenden Ansprüche, insbesondere zur Verwendung als Badezusatz,
**dadurch gekennzeichnet, dass**
es eine saure, puffernde Lösung umfasst, insbesondere im pH-Bereich von 1,5 bis 6 und dabei bevorzugt einen Gehalt an Fruchtsäueren und/oder Fruchtsalzen und/oder Carbonat/Bicarbonat und/oder Phosphat sowie mindestens ein Metall aus der Gruppe enthält, die Eisen, Kupfer, Chrom, Mangan, Zink umfasst, wobei dieser vorzugsweise 1 bis 0,5 Gew.% nicht überschreitet.

37. Präparat nach einem der vorhergehenden Ansprüche,
**dadurch gekennzeichnet, dass**
es als isotone Lösung insbesondere für Injektionen vorliegt.

38. Präparat nach einem der vorhergehenden Ansprüche,
**dadurch gekennzeichnet, dass**
der Breitbandresonator und/oder der oder die Wirkstoffe zur Vermeidung von Wechselwirkungen in Liposomen eingebracht sind.

39. Präparat nach einem der vorhergehenden Ansprüche, insbesondere zur Behandlung von Rheuma,
**dadurch gekennzeichnet, dass**
als Wirkstoff ein oder mehrere Stoffe aus der Gruppe Bryonia, Rhus toxicodendron, Thuja, Carduus marianus, Cheledonium, Nux vomica, Spiraea ulmaria, Dulcamara, Clematis, Veratrum album, Sulfur, Staphisagria enthalten sind, wobei diese vorzugsweise im fertigen Präparat in der Konzentration von der Urtinktur bis D12 enthalten sind, vorzugsweise in Verbindung mit einer Resonatorenkonzentration im Bereich von D1 bis D12..

40. Präparat nach einem der vorhergehenden Ansprüche, insbesondere zur Behandlung von Lebererkrankungen,
**dadurch gekennzeichnet, dass**
als Wirkstoff mindestens eine Komponente aus der Gruppe, die Cheledonium, Carduus marianus, Mandragora officinarium und/oder Leptandara, in einer Konzentration von der Urtinktur bis D 12 vorzugsweise in Verbindung mit einer Resonatorenkonzentration im Bereich von D1 bis D15, vorzugsweise D2 bis D4 vorgesehen ist.

41. Präparat nach einem der vorhergehenden Ansprüche, insbesondere zur Wundbehandlung,
**dadurch gekennzeichnet, dass**
es als Inhaltsstoffe Himalajasalz/Steinsalz als Resonator, insbesondere in der Konzentration D8, eine saure Spurenelement-Lösung, insbesondere in der Konzentration D3 bis D4, Vitamine A und/oder B und/oder C, Panthothensäure, eine Emulsion als O/W - und/oder W/O - Formulierung sowie Grapefruitkernextrakt umfasst.

42. Mehrstufenpräparat zur Durchführung einer mehrstufigen Therapie mit mehreren der Präparate nach einem der vorhergehenden Ansprüche, bei denen jedes eine unterschiedliche Konzentration an dem oder den Resonatoren und/oder ein unterschiedliches Polaritäts/Resonatorenkonzentrations-Verhältnis und/oder eine unterschiedliche Polarität aufweist.

43. Mehrstufenpräparat nach Anspruch 42,
**dadurch gekennzeichnet, dass**
ein erstes Präparat zur Durchführung der ersten Stufe einer Therapie vorgesehen ist, welches eine niedrige Konzentration an dem oder den Resonatoren und/oder ein hohes Polaritäts/Resonatorenkonzentrations-Verhältnis aufweist und/oder eine hohe Polarität aufweist, und mindestens ein weiteres, vorzugsweise mehrere weitere Präparate zur Durchführung weiterer Stufen einer Therapie vorgesehen sind, welche jeweils höhere Konzentrationen an dem oder den Resonatoren und/oder niedrigere Polaritäts/Resonatorenkonzentrations-Verhältnisse und/oder niedere Polaritäten aufweisen.

44. Präparate-Set, insbesondere zur Durchführung einer zweigleisigen Therapie mit zwei oder mehr Präparaten nach einem der Ansprüche 1 bis 43,
**dadurch gekennzeichnet, dass**
es ein Plus ― Präparat mit einer sauren polaren Trägersubstanz und ein Minus - Präparat mit einer basischen polaren Trägersubstanz aufweist.

45. Präparate-Set nach Anspruch 44, insbesondere zur Verwendung an Akkupunkturpunkten, Chakren und/oder Meridianen,
**dadurch gekennzeichnet, dass**
es mit mindestens ein Minus- und ein Plus-Präparat enthält und als Flüssig- und/oder Halbflüssigpräparat, bevorzugt als Creme, Lotion oder Salbe formuliert ist.

46. Präparate-Set nach Anspruch 44 oder 45,
**dadurch gekennzeichnet, dass**
es mineralische und pflanzliche Wirkstoffe enthält und vorzugsweise 2 mal 3 bis 2 mal 7 Salbenpräparate umfasst.

47. Verfahren zur Herstellung eines Präparats nach einem der Ansprüche 1 bis 46,
**dadurch gekennzeichnet, dass**
ein homöopathischen Potenzierungsverfahrens, insbesondere zur Erzeugung einer Niedrig-, Mittel- oder Hochpotenz, vorzugsweise Dezimalpotenz oder Centesimalpotenz und/oder ein Energetisierungsverfahren, insbesondere gemäß dem Bioresonanz- und/oder Magnetisierungsverfahren durchgeführt wird.

48. Verfahren nach Anspruch 47,
**dadurch gekennzeichnet, dass**
die Wechselwirkung zwischen spezifischen Wirkstoff(en) und dem oder den Resonatoren und/oder die energetischen Verhältnisse der Gewebe und Strukturen berücksichtigt werden.

49. Verfahren nach Anspruch 45 oder 46,
**dadurch gekennzeichnet, dass**
die Resonatorkonzentration der Wirkstoffkonzentration angepasst wird, insbesondere im Bereich von wenigen Dezimalpotenzen über oder unter der Wirkstoffpotenz.

50. Verfahren nach einem der Ansprüche 27 bis 29,
**dadurch gekennzeichnet, dass**
die Resonatorkonzentration bei nicht homöopathischen Wirkstoffen umso höher gewählt wird, je toxischer der Wirkstoff ist und bei homöopathischen Wirkstoffen umso niedriger.

51. Verfahren nach einem der Ansprüche 27 bis 30,
**dadurch gekennzeichnet, dass**
die Polarität umso höher eingestellt wird, je höher die Konzentration des Resonators ist.

52. Verfahren nach einem der Ansprüche 27 bis 31,
**dadurch gekennzeichnet, dass**
ein elektrolytisches Verfahren eingesetzt wird.

53. Verfahren nach einem der Ansprüche 45 bis 50,
**dadurch gekennzeichnet, dass**
bei basischen Komponenten oder Minuskomponenten auf die Verwendung von Metallen oder Verbindungen, die Eisen, Kupfer, Chrom oder Mangan umfassen, verzichtet wird.

54. Verfahren nach einem der Ansprüche 45 bis 50,
**dadurch gekennzeichnet, dass**
der Wirkstoff und/oder der Breitbandresonator und/oder die polare Trägersubstanz getrennt voneinander verdünnt und/oder einem homöopathischen Potenzierungsverfahren unterzogen werden und danach wieder zu einem Präparat zusammengeführt werden, wobei bevorzugt eine homöopathische Aufbereitung (Verschüttelung) durchgeführt wird.

55. Verfahren nach einem der Ansprüche 45 bis 50,
**dadurch gekennzeichnet, dass**
die Ausgangslösungen (homöopathischen Urtinkturen) von Breitbandresonator und polarer Trägersubstanz die gleiche Polarität ggf. in unterschiedlicher Ausprägung aufweisen.

56. Verfahren nach einem der Ansprüche 45 bis 50,
**dadurch gekennzeichnet, dass**
die Ausgangslösungen (homöopathischen Urtinkturen) von Breitbandresonator und polarer Trägersubstanz einen unterschiedlichen pH-Wert und/oder eine unterschiedliche Polarität aufweisen.

57. Verfahren nach einem der Ansprüche 45 bis 50,
**dadurch gekennzeichnet, dass**
der Wirkstoff und/oder der Breitbandresonator und/oder die polare Trägersubstanz getrennt voneinander in unterschiedlichem Maße verdünnt und/oder potenziert werden, wobei der Verdünnungsgrad jeweils auf einem anderen Algorithmus beruht, und zwar vorzugsweise so, dass dies mit jedem Verdünnungs- und/oder Potenzierungsschritt zu einer relativen stärkeren Verdünnung des Breitbandresonators führen.

58. Verfahren nach einem der Ansprüche 45 bis 50,
**dadurch gekennzeichnet, dass**
der Breitbandresonator und die polaren Trägersubstanz getrennt voneinander in unterschiedlichen Schritten verdünnt werden, wobei die Differenz der Konzentration der enthaltenen Stoffe mit zunehmender Verdünnung vorzugsweise in nichtlinearer Weise zu oder abnimmt.

59. Verfahren nach einem der Ansprüche 45 bis 50,
**dadurch gekennzeichnet, dass**
zuerst ein basischer pH-Wert eingestellt wird, der dann mittels Zugabe von Säure neutralisiert wird.

60. Verwendung eines Präparats nach einem der Ansprüche 1 bis 26 zur Herstellung einer insbesondere arzneilich wirkenden Substanz zur Unterstützung der Aufnahme und/oder Steigerung der Wirkung von Wirkstoffen, insbesondere arzneilichen Wirkstoffen, oder Nahrungsergänzungsmitteln in einem menschlichen oder tierischen Körper, insbesondere unter Vermeidung von Nebenwirkungen und/oder zur Verringerung oder Vermeidung von schädlichen Wirkungen von auf den menschlichen oder tierischen Körper einwirkender Stoffe, wie Kosmetika und dergleichen.

61. Verwendung eines Präparats nach einem der Ansprüche 1 bis 26 zur Herstellung einer Substanz zur Diagnose mittels eines Informierungs- und/oder Energetisierungs- und/oder Aufschwingungsverfahrens, insbesondere gemäß einem Bioresonanzverfahren und/oder mittels eines radionischen Verfahrens unter Verwendung eines Bioresonanzgeräts und/oder mittels eines Magnetisierungsverfahrens.
